# EUROPEAN PATENT SPECIFICATION

(11) **EP 2 536 845 B1**
(45) Date of publication and mention of the grant of the patent: **21.01.2015**
(21) Application number: 11704846.2
(22) Date of filing: 15.02.2011
(51) Int. Cl.: C12Q 1/68

(54) **OLIGONUCLEOTIDE BASED ANALYTE DETECTION METHOD**
AUF OLIGONUKLEOTIDEN BASIERENDES VERFAHREN FÜR DEN NACHWEIS VON ANALYTEN
PROCÉDÉ DE DÉTECTION D'ANALYTE À BASE D'OLIGONUCLÉOTIDE

(30) Priority: 16.02.2010 GB 201002627
(43) Date of publication of application: 26.12.2012
(73) Proprietor: Loxbridge Research LLP, London W1S 4BS (GB)
(72) Inventor: KOJIC, Igor, London W1S 4BS (GB); ROBERTS, Charles Edward Selkirk, London W1S 4BS (GB)
(74) Representative: Gibson, Mark
(86) International application number: PCT/GB2011/050295
(87) International publication number: WO 2011/101666

(56) References cited:
- WO-A2-2006/119308
- WO-A2-2009/104075
- US-A- 5 981 297
- US-A1- 2006 257 958
- JOHN G BRUNO ET AL: "Plastic-Adherent DNA Aptamer-Magnetic Bead and Quantum Dot Sandwich Assay for Campylobacter Detection", JOURNAL OF FLUORESCENCE, KLUWER ACADEMIC PUBLISHERS-PLENUM PUBLISHERS, NE, vol. 19, no. 3, 4 December 2008 (2008-12-04), pages 427-435, XP019681028, ISSN: 1573-4994
- PYUN J C ET AL: "Development of a biosensor for E. coli based on a flexural plate wave (FPW) transducer", BIOSENSORS AND BIOELECTRONICS, ELSEVIER BV, NL, vol. 13, no. 7-8, 1 October 1998 (1998-10-01), pages 839-845, XP002342141, ISSN: 0956-5663, DOI: DOI:10.1016/S0956-5663(98)00050-5
- CENTI SONIA ET AL: "Aptamer-based detection of plasma proteins by an electrochemical assay coupled to magnetic beads.", ANALYTICAL CHEMISTRY 15 FEB 2007 LNKD- PUBMED:17297945, vol. 79, no. 4, 15 February 2007 (2007-02-15), pages 1466-1473, XP002635198, ISSN: 0003-2700
- PAN Y ET AL: "Selective collection and detection of leukemia cells on a magnet-quartz crystal microbalance system using aptamer-conjugated magnetic beads", BIOSENSORS AND BIOELECTRONICS, ELSEVIER BV, NL, vol. 25, no. 7, 2 December 2009 (2009-12-02), pages 1609-1614, XP026905665, ISSN: 0956-5663, DOI: DOI:10.1016/J.BIOS.2009.11.022 [retrieved on 2009-12-02]
- Sonia Centi ET AL: "Detection of C Reactive Protein (CRP) in Serum by an Electrochemical Aptamer-Based Sandwich Assay", Electroanalysis, vol. 21, no. 11, 1 June 2009 (2009-06-01), pages 1309-1315, XP055124841, ISSN: 1040-0397, DOI: 10.1002/elan.200804560

## Description

### FIELD OF THE INVENTION

The invention relates to a method of detecting the presence of an analyte within a sample, wherein said method comprises an oligonucleotide based approach. The invention also relates to methods of diagnosing diseases, in particular, but not exclusively infectious diseases such as septicaemia.

### BACKGROUND OF THE INVENTION

The detection of analytes, such as biomolecules, for example proteins, can be highly beneficial in the diagnosis of diseases or medical conditions. By determining the presence of a specific protein or peptides associated with a specific protein, investigators can confirm the presence of a virus, bacterium, genetic mutation, or other condition that relates to a disease-state. Furthermore, by analyzing a patient's proteome, i.e., the patient's unique set of expressed proteins, useful information relating to an individual's need for particular medicines or therapies can be determined, so as to customize a course of treatment or preventative therapy.

Current methods for detecting analytes include simple methods such as Western blot analysis, Immunochemical assay, and enzyme-linked immunosorbent assay (ELISA). There are more recent methods still using piezoelectric technology to detect the analyte levels (such as those described in WO 2006/119308 and WO 2007/030155).

However, there is a need for a new method of detecting organic molecules in samples, for instance human serum, as a tool to be used in the diagnosis of disorders, for instance septicaemia. Furthermore, there is a need for improved analyte detection methods which are capable of detecting much lower levels of analyte, are much faster, and more accurate.

### SUMMARY OF THE INVENTION

According to a first aspect of the invention there is provided a method of detecting the presence of an analyte within a sample, wherein said method comprises the steps of:
(a) preparing one or more magnetic complexes comprising a magnetic particle coated with a first binding agent capable of specific binding to the analyte, wherein a spacer element is present between the first binding agent and the magnetic particle;
(b) adding the one or more magnetic complexes prepared in step (a) to the sample within a receptacle, wherein a surface of a receptacle is coated with a second binding agent capable of specific binding to the analyte, such that at least one of said first and second binding agents is a DNA or RNA oligonucleotide;
(c) temporarily applying a magnetic field to the surface of the receptacle which is coated with the second binding agent; and
(d) detecting the presence of magnetic complexes which remain bound to the surface of the receptacle following step (c) which is indicative of the presence of the analyte within the sample.

According to a second aspect of the invention there is provided a method of diagnosing or monitoring a disease within a subject, wherein said diagnostic method comprises the steps of:
(a) preparing one or more magnetic complexes comprising a magnetic particle coated with a first binding agent capable of specific binding to an analyte biomarker which is characteristic for the disease, wherein a spacer element is present between the first binding agent and the magnetic particle;
(b) adding the one or more magnetic complexes prepared in step (a) to a biological sample obtained from the subject within a receptacle, wherein a surface of a receptacle is coated with a second binding agent capable of specific binding to the analyte biomarker which is characteristic for the disease, such that at least one of said first and second binding agents is a DNA or RNA oligonucleotide;
(c) temporarily applying a magnetic field to the surface of the receptacle which is coated with the second binding agent; and
(d) detecting the presence of magnetic complexes which remain bound to the surface of the receptacle following step (c) which is indicative of the presence of the analyte biomarker characteristic for the disease within the biological sample, or a binding value which is above or below a threshold value characteristic for the disease may be indicative of presence of the disease or risk for the disease.

According to a further aspect of the invention there is provided an analyte detection kit which may comprise one or more magnetic complexes comprising a magnetic particle and/or surface coated with a DNA or RNA oligonucleotide capable of specific binding to the analyte. This kit may be provided as a disposable 'chip' to be used with the relevant hardware.

### BRIEF DESCRIPTION OF THE FIGURES

**Figures 1 to 4** show schematic representations of selected embodiments of the method of the invention; and
**Figures 5 to 10** describe the results of detecting thrombin in a ViBE Bioanalyzer in accordance with the methods of the invention.

### DETAILED DESCRIPTION OF THE INVENTION

According to a first aspect of the invention there is provided a method of detecting the presence of an analyte within a sample, wherein said method comprises the steps of:
(a) preparing one or more magnetic complexes comprising a magnetic particle coated with a first binding agent capable of specific binding to the analyte, wherein a spacer element is present between the first binding agent and the magnetic particle;
(b) adding the one or more magnetic complexes prepared in step (a) to the sample within a receptacle, wherein a surface of a receptacle is coated with a second binding agent capable of specific binding to the analyte, such that at least one of said first and second binding agents is a DNA or RNA oligonucleotide;
(c) temporarily applying a magnetic field to the surface of the receptacle which is coated with the second binding agent; and
(d) detecting the presence of magnetic complexes which remain bound to the surface of the receptacle following step (c) which is indicative of the presence of the analyte within the sample.

The method of the invention provides a fast and effective technique for detecting extremely low levels of an analyte within a sample. The method of the invention therefore finds great utility primarily in the pharmaceutical fields of diagnosis and prognosis, as well as in the fields of environmental and quality control, i.e. detection of contaminants.

Some ~30-mer single-stranded oligonucleotides display binding properties (high specificity and affinity) to certain organic molecules (for instance peptides), in which case they have been termed 'aptamers'. Importantly, aptamers do not bind due to complement nucleotide binding, but rather due to their tertiary structure. References herein to DNA or RNA oligonucleotides may be used interchangeably with the term aptamer.

Aptamers provide a number of advantages over antibody based analyte detection procedures. For example, aptamers can be generated and selected in the laboratory relatively quickly, without the need for the maintenance costs and ethical approvals for animal procedures which are required for the raising of polyclonal or monoclonal antibodies by conventional procedures. This is especially relevant where the analyte is a molecule that has toxic properties, or is otherwise harmful, to a host organism used for raising antibodies. Additionally, aptamers can be produced relatively cheaply in the required, and potentially unlimited quantities, readily and in pure form uncontaminated by biological material, using existing synthetic procedures.

Without being bound by theory, the method of the invention is believed to work by forming an analyte-first binding agent-magnetic particle complex in the presence of an analyte within a sample. Upon application of the magnetic field in step (c), the analyte-first binding agent-magnetic particle complex will be attracted to the surface of the receptacle which is coated with the second binding agent. The presence of the second binding agent additionally on the surface of the receptacle ensures that the analyte-first binding agent-magnetic particle will remain tightly bound to the surface of the receptacle even after the magnetic field has been removed (i.e. a second binding agent -analyte-first binding agent-magnetic particle complex will be formed on the surface of the receptacle). This provides a significant advantage of allowing differentiation between attraction of the analyte-first binding agent-magnetic particle to the surface of the receptacle and attraction of unbound first binding agent-magnetic particles which will also be attracted to the surface of the receptacle by the magnetic field.

The concept of applying a magnetic field to attract magnetic particles and subsequent detection steps are well known in the art and are described in detail in WO 2006/119308 and WO 2007/030155. For example, the magnetic field is believed to induce a polarization in the magnetic material of the particle that is aligned with the local magnetic field lines. The particle experiences a net force in the direction of the gradient, causing the particle to migrate toward regions of higher field strength. The magnetic field distribution is configured to attract analyte-first binding agent-magnetic particle complexes from the sample and distribute them across the surface of the receptacle. The background components of the sample, such as cells, reagents and proteins, generally have a much lower magnetic susceptibility as compared to the magnetic particles, and so the magnetic field does not significantly influence them. Hence, only a very small fraction of this background material interacts with the receptacle surface.

In one embodiment, the sensing device comprises a piezoelectric sensing device. Such a piezoelectric sensing device functions by using a piezoelectric effect to measure pressure exerted by binding of the magnetic particles to the surface of the receptacle and converting the pressure signal to an electrical signal. The resultant electrical signal will provide a direct value of the degree of binding of the magnetic particles to the surface of the receptacle.

Examples of piezoelectric sensing devices include those described in WO 2006/119308 and WO 2007/030155. For example, in one embodiment, the surface of the receptacle comprises a sensing device, such as an acoustic device. Acoustic devices couple to fluids predominantly through acoustic interaction between the device and the fluid. Typical acoustic devices include surface acoustic wave devices, flexural plate wave devices, lamb wave devices and cantilever devices. Acoustic devices also couple to fluids through some viscous interaction between the device and the fluid, however, the coupling is predominantly acoustic coupling. Viscous interaction devices couple to fluids predominantly through viscous interaction between the devices and the fluid. Typical viscous interaction devices include quartz microbalance (QCM) devices, shear harmonic surface acoustic wave devices, and acoustic plate mode devices. The term "surface acoustic wave" refers to the manner in which energy is carried in the device structure rather than how the device couples to the fluid. Acoustic devices are devices where fluid interacts over a substantial area of a plane of the device. Acoustic devices respond with substantial out of plane motion that couples acoustically to fluid in proximity to the plane of the device (i.e. kinetic energy, potential energy and losses are carried predominantly in the fluid). Viscous interaction devices respond primarily with in-plane motion that does not couple acoustically to fluid in proximity to a plane of the device.

In one embodiment, the acoustic device is a flexural plate wave (FPW) device. The presence of an FPW device provides a number of advantages. Firstly, the presence of the magnetic particles on the surface of the receptacle results in an amplified FPW signal response which may be detected by a monitoring device configured to monitor the signal output from the FPW device. The larger combined size and density of the analyte-first binding agent-magnetic particle complex yields a larger FPW signal response than the analyte alone. Secondly, the surface of the sensor in the FPW device consists of a thin membrane that is typically only a few micrometers thick, which allows larger magnetic fields and field gradients to be created at the sensor surface because the field source can be positioned closer to the sample flow. This results in enhancement of the attraction of magnetic particles to the surface of the receptacle and therefore higher fractional capture of the analyte from the sample. With this higher capture rate and efficiency, it is possible to process larger sample volumes in shorter times than would be otherwise possible.

Further examples of piezoelectric sensing devices include those described in US 2009/293590, US 2009/168068, WO 2009/035732, US 2009/282902, WO 2008/019694, US 2006/105471, US 2006/014270, US 2005/186684, WO 2004/001392, WO 2004/001417, WO 2004/001416, WO 01/26138, US 6,161,426, US 5,447,845 and DE 4216980.

In an alternative embodiment, the sensing device comprises an optical sensing device.

Examples of suitable optical sensing devices comprise those which require flow cytometry which utilizes an optical technique that analyzes particles in a fluid mixture based on the particles' optical characteristics using a flow cytometer. Flow cytometers hydrodynamically focus a fluid suspension of particles into a thin stream so that the particles flow down the stream in substantially single file and pass through an examination zone. A focused light beam, such as a laser beam illuminates the particles as they flow through the examination zone. Optical detectors within the flow cytometer measure certain characteristics of the light as it interacts with the particles. Commonly used flow cytometers such as the Becton-Dickinson Immunocytometry Systems "FACSCAN" can measure forward light scatter (generally correlated with the refractive index and size of the particle being illuminated), side light scatter (generally correlated with the particle's size), and particle fluorescence at one or more wavelengths. (Fluorescence is typically imparted by incorporating, or attaching a fluorochrome within the particle.) Flow cytometers and various techniques for their use are described in, generally, in "Practical Flow Cytometry" by Howard M. Shapiro (Alan R. Liss, Inc., 1985) and "Flow Cytometry and Sorting, Second Edition" edited by Melamed et al. (Wiley-Liss, 1990). The person skilled in the art will recognize that one type of "particle" analyzed by a flow cytometer may be man-made microspheres or beads, such as the magnetic particles of the present invention.

Examples of suitable flow cytometry based optical sensing methods are described in WO 99/58955, US 2003/132538, US 2005/118574, US 2002/096795, WO 01/13119, US 6,524,793, WO 99/36564, US 6,449,562, US 6,057,107 and US 5,981,180.

A further example of a suitable optical sensing device comprises the use of a chromophore (i.e. fluorescein), such that the second binding agent may be conjugated to a signaling chromophore such that upon excitation is capable of transferring energy to the sensor signaling chromophore. The sample containing the analyte will typically be contacted with the second binding agent and the multichromophore in a solution under conditions in which the second binding agent can bind to the analyte if present. The process would then require the step of applying a light source to the sample that can excite the multichromophore, and detecting whether light is emitted from the signaling chromophore which would be indicative of the presence of an analyte in the sample. One such example of a chromophore based optical sensing approach is described in WO 2008/100344.

It will be appreciated that the concentration of analyte in the sample is calculated using analysis algorithms using the signal output from the sensing device as source data. Thus, in one embodiment, the signal output of the sensing device within the receptacle (i.e. the FPW device) in response to sample exposure is compared to or normalized with a control signal. The control signal can be provided to or obtained by the user. For example, the control signal can be a value provided to the user based on the specific analyte, binding agent, or particular model, version or type of device being used. In one embodiment, the control signal is obtained on a lot basis. For example, the control signal can be a signal that is representative of a particular lot of analyte acquired by the user. The representative signal can be, for example, experimentally derived before, during or after testing of a sample. In some embodiments, the control signal is a standard curve that is obtained by, for example, analyzing known quantities of analyte with a specific binding agent and specific version of a sensing device. In another embodiment, the control signal is obtained on a use basis. For example, a unique control signal can be obtained each time a particular analyte and/or binding agent is tested on a particular sensing device.

It will also be appreciated that the sensing device present within the surface of the receptacle coated with second binding agent may be configured to measure and/or detect the weight change associated with binding of the analyte-first binding agent-magnetic particle complexes.

As discussed hereinbefore, it will be appreciated that application of the magnetic field in step (c) will result in attraction of all magnetic particles within the sample to the surface of the receptacle regardless of whether they contain bound analyte. Therefore, in one embodiment the method of the invention additionally comprises a wash step following application of the magnetic field in step (c). This wash step provides the advantage of removing any first binding agent-magnetic particle complexes which do not contain analyte along with any other non-specifically bound components. In one embodiment, the detection step (d) is performed after the magnetic field has been removed. This embodiment provides the advantage of allowing any magnetic particles not bound to an analyte to be removed from the surface of the receptacle. In a further embodiment, the detection step (d) is performed after the wash step. In a yet further embodiment, the detection step (d) is performed after the magnetic field has been removed and after the wash step.

It will be appreciated that the presence of at least one DNA or RNA oligonucleotide (i.e. aptamer) on the magnetic particle or coated upon a surface of the receptacle constitutes a significant departure from previous detection methods. Aptamers are synthetic, highly structured, single-standed DNA or RNA ligands. Aptamers have two important properties, namely the ability to fold into complex tertiary structures and to bind with high affinity (low nM to high pM equilibrium dissociation constants) and specificity to their targets. Importantly, aptamers do not bind due to complement nucleotide binding, but rather due to their tertiary structure. Aptamers are a relatively little-known, and expanding, field of interest. They have advantages over many other types of binding agent because of (i) their "programmability" (since the selection process starts with so many options, an aptamer can almost always be found to bind a chosen molecule), (ii) their wide range of applicable types of target (protein, peptide, monomer, and many others, for many of which there is no other binding agent in existence or readily conceivable).

The sequence of the aptamer of the invention may be selected by any method known in the art. In one embodiment, the aptamer may be selected by an iterative selection process such as Systemic Evolution of Ligands by Exponential Enrichment (SELEX). In this type of process, a random pool of oligonucleotides (e.g., about 10⁵ to about 10¹⁷ random oligonucleotides) is exposed to a target analyte (i.e. target protein) and the oligonucleotides that bind to the target are then specifically recovered. These sequences are amplified with PCR or RT-PCR. Single stranded RNA or DNA sequences representing the recovered sequences are then generated from these PCR products, and the process is repeated until oligonucleotides that bind with the desired affinity to the target are identified. It will be appreciated that other aptamer selection methods may also be employed such as those invented subsequently to the SELEX process. Thus, in an alternative embodiment the DNA or RNA oligonucleotide is selected by bead based selection methods, capillary electrophoresis, or selection methods using microfluidics.

References herein to "specific binding", refers to an aptamer that binds to an analyte with at least five-fold greater affinity as compared to any non-targets, e.g., at least 10-, 20-, 50-, or 100-fold greater affinity.

The length of the aptamers of the invention is not limited, but typical aptamers have a length of about 10 to about 100 nucleotides, e.g., about 20 to about 80 nucleotides, about 30 to about 50 nucleotides, or about 40 nucleotides. In certain embodiments, the aptamer may have additional nucleotides attached to the 5'- and/or 3' end. The additional nucleotides may be, e.g., part of primer sequences, restriction endonuclease sequences, or vector sequences useful for producing the aptamer.

The polynucleotide aptamers of the present invention may be comprised of ribonucleotides only (RNA aptamers), deoxyribonucleotides only (DNA aptamers), or a combination of ribonucleotides and deoxyribonucleotides. The nucleotides may be naturally occurring nucleotides (e.g., ATP, TTP, GTP, CTP, UTP) or modified nucleotides. Modified nucleotides refers to nucleotides comprising bases such as, for example, adenine, guanine, cytosine, thymine, and uracil, xanthine, inosine, and queuosine that have been modified by the replacement or addition of one or more atoms or groups. Some examples of types of modifications that can comprise nucleotides that are modified with respect to the base moieties, include but are not limited to, alkylated, halogenated, thiolated, dithiolated, aminated, amidated, or acetylated bases, in various combinations. More specific examples include 5- propynyluridine, 5-propynylcytidine, 6-methyladenine, 6-methylguanine, N5N,-dimethyladenine, 2-propyladenine, 2-propylguanine, 2-aminoadenine, 1 -methylinosine, 3-methyluridine, 5-methylcytidine, 5-methyluridine and other nucleotides having a modification at the 5 position, 5-(2-amino)propyl uridine, 5-halocytidine, 5-halouridine, 4- acetylcytidine, 1 -methyladenosine, 2-methyladenosine, 3-methylcytidine, 6-methyluridine, 2- methylguanosine, 7-methylguanosine, 2,2-dimethylguanosine, 5-methylaminoethyluridine, 5- methyloxyuridine, deazanucleotides such as 7-deaza-adenosine, 6-azouridine, 6-azocytidine, 6-azothymidine, 5-methyl-2-thiouridine, other thio bases such as 2-thiouridine and 4- thiouridine and 2-thiocytidine, dihydrouridine, pseudouridine, queuosine, archaeosine, naphthyl and substituted naphthyl groups, any O- and N-alkylated purines and pyrimidines such as N6-methyladenosine, 5-methylcarbonylmethyluridine, uridine 5-oxyacetic acid, pyridine-4-one, pyridine-2-one, phenyl and modified phenyl groups such as aminophenol or 2,4,6-trimethoxy benzene, modified cytosines that act as G-clamp nucleotides, 8-substituted adenines and guanines, 5-substituted uracils and thymines, azapyrimidines, carboxyhydroxyalkyl nucleotides, carboxyalkylaminoalkyl nucleotides, and alkylcarbonylalkylated nucleotides. Modified nucleotides also include those nucleotides that are modified with respect to the sugar moiety (e.g., 2'-fluoro or 2'-O-methyl nucleotides), as well as nucleotides having sugars or analogs thereof that are not ribosyl. For example, the sugar moieties may be, or be based on, mannoses, arabinoses, glucopyranoses, galactopyranoses, 4'-thioribose, and other sugars, heterocycles, or carbocycles. The term nucleotide is also meant to include what are known in the art as universal bases. By way of example, universal bases include but are not limited to 3-nitropyrrole, 5 -nitro indole, or nebularine. Modified nucleotides include labeled nucleotides such as radioactively, enzymatically, or chromogenically labeled nucleotides). In one particular embodiment, the DNA or RNA oligonucleotides are thiolated or dithiolated. Such thiolation provides the advantage of ensuring that the oligonucleotide is resistant to endonuclease activity.

In a further particular embodiment, the DNA or RNA oligonucleotides comprise spiegelmers. Spiegelmers are oligonucleotide mirror images (L-form nucleotides), which due to their optical re-configuration confer resistance to endonuclease activity whilst maintaining specificity of the 'regular' (D-form) type of oligonucelotides. Spiegelmers are well characterized and have been shown to demonstrate high affinity for specific targets, such as gonadotrophin (Wlotzka et al, PNAS June 25, 2002 vol. 99 no. 13 8898-8902).

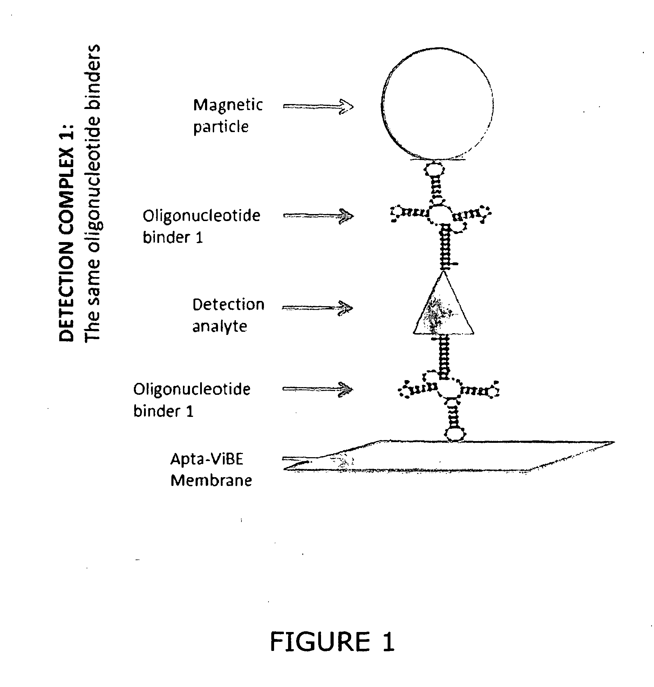

It will be appreciated that the first binding agent used to form the magnetic complex and the second binding agent used to coat the surface of the receptacle must both bind specifically to the analyte, however, the physical composition of the two binding agents may be different. In one embodiment, the first binding agent used to form the magnetic complex is identical to the second binding agent used to coat the surface of the receptacle. References to "identical" include greater than any one of 80, 85, 90, 91, 92, 93, 94, 95, 96, 97, 98, 99 or 100% sequence identity or any ranges therebetween. A schematic representation of the embodiment where the first and second binding agents are identical may be seen in Figure 1. It will be appreciated that other binding moieties (e.g. those referred to herein as first and second binding pairs, such as biotin/strepavidin complexes) may be present between any of the binding agents and the magnetic particle or membrane to which they are bound.

In one embodiment, the first and second binding agents are both DNA or RNA oligonucleotides (i.e. both aptamers).

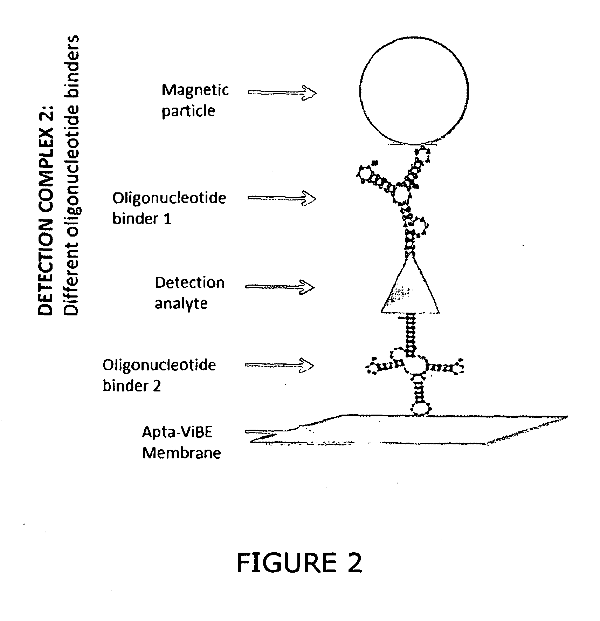

In an alternative embodiment, the first binding agent differs from the second binding agent. For example, the first and second binding agents may both be DNA or RNA oligonucleotides having differing sequences. A schematic representation of the embodiment where the first and second binding agents are both DNA or RNA oligonucleotides having differing sequences may be seen in Figure 2. It will be appreciated that other binding moieties (e.g. those referred to herein as first and second binding pairs, such as biotin/strepavidin complexes) may be present between any of the binding agents and the magnetic particle or membrane to which they are bound.

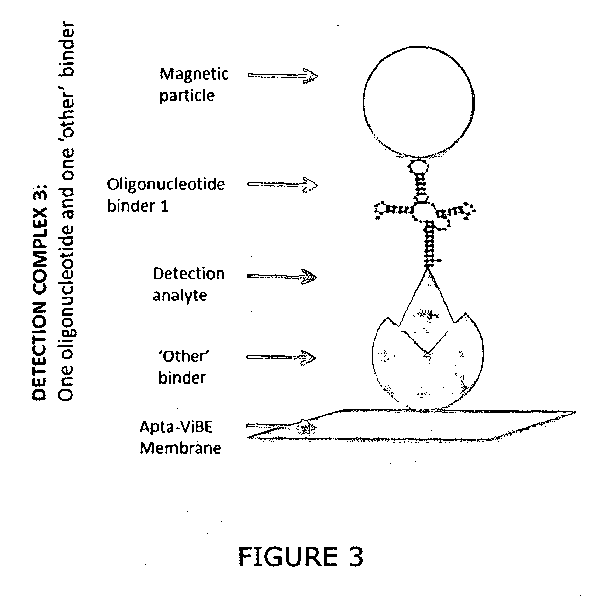

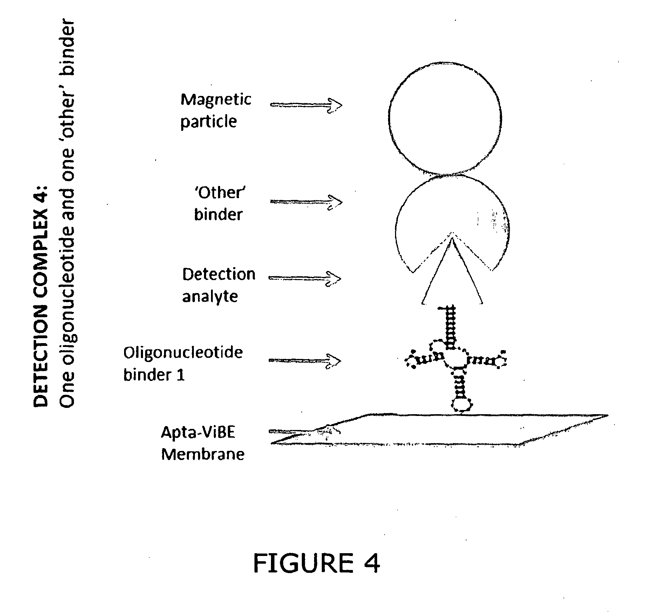

Alternatively, one of the first and second binding agents may be a DNA or RNA oligonucleotide and the other may be an antibody. Schematic representations of the embodiment where one of the first and second binding agents may be a DNA or RNA oligonucleotide and the other may be an alternative binding molecule such as an antibody may be seen in Figures 3 and 4. It will be appreciated that the term "Other" used in Figures 3 and 4 may also refer to a peptide, or any other binding molecule, as well as an antibody.

In one embodiment the magnetic particle comprises a magnetic bead. The use and availability of such magnetic beads is known to the person skilled in the art, a non-limiting example of which includes Dynabeads^{RTM} (Invitrogen, UK). In one embodiment the particles are added to the sample in a concentration from about 1 x 10² to about 1 x 10⁷ per ml. In a further embodiment, the particles are added to the sample in a concentration from about 5 x 10³ to about 5 x 10⁵ per ml.

Binding agents can be bound to the surface of the receptacle or to the magnetic bead in accordance with standard techniques for attaching nucleic acids, and the like to surfaces.

In one embodiment, binding agents are directly bound to the surface of the receptacle or the magnetic bead.

In an alternative embodiment, the binding agents are indirectly bound to the surface of the receptacle or the magnetic bead. The binding agent can be indirectly bound to the surface of the receptacle or the bead, for example, by coating the surface or bead with a first member of a binding pair. The binding agent is bound or attached to a second member of the binding pair and then the binding agent is bound to the surface or the bead via the interaction between the first and second members of the binding pair. Suitable binding pairs include, for example, biotin and avidin or biotin and derivatives of avidin such as streptavidin and neutravidin.

In one embodiment, the first member of a binding pair comprises an antibody or RNA or DNA oligonucleotide and the second member of the binding pair comprises the first or second binding agents.

In an alternative embodiment, the second binding agent may be indirectly attached to the surface of the receptacle via a fluorophore, such as fluoroscein.

In one embodiment, the spacer element comprises a triethylene glycol (TEG) spacer element, such as a triethylene glycol 16-atom mixed polarity spacer. Data is presented herein which shows that excellent detection results were obtained for thrombin in the presence of such a TEG spacer element.

Organic molecules like proteins, DNA, and RNA have binding regions or sites to which specific molecules and ions, called ligands, can bind to or associate. Within these binding regions, interactions are principally stabilized by chemical bond associations, which vary in affinity depending on the interacting molecules present. Binding site competition can arise when more than one type of ligand is attempting to associate to the same binding site. Many organic molecules vary in the number of binding regions present, and in the case of human alpha thrombin there are three binding sites, which exhibit different specificities. Human alpha thrombin has an active site that shows equal affinity for both fibrin as for fibrinogen. Human alpha thrombin also has two patches of positive surface potential that mediate intermolecular interactions: the fibrinogen-recognition exosite (also termed anion-binding exosite I) and the heparin-binding exosite (or anion-binding exosite II). Enzymes, like human alpha thrombin, that have an exosite as a secondary binding site, require that the exosite be occupied in order for the enzyme to be active. In instances where the current embodiment (which needs an analyte to have two binding sites, to which two different aptamers are engineered with affinity) requires the detection of a molecule that has only one active site where only one aptamer has been engineered a specificity to, then a modified version of the detection method must be applied in order to facilitate the required detection complex pathway. The bi-functional aptamer approach utilizes an aptamer that is functionalized with biotin at one end (5' or 3') and a fluorescein at the other end (5' or 3') and exhibits specificity for the only available active site on the organic molecule or analyte. A TEG carbon-spacer linker can also be used (either on the 5' or 3' ends), as a means to extend the aptamer for the purposes outlined below. The bi-functional aptamer is pre-mixed with the magnetic beads and test solution containing the analyte of interest before being introduced into the Apta-ViBE. The resulting complex (aptamer bound to magnetic bead and tangentially associated with the analyte of interest) is then established upon the Apta-ViBE detection membrane and signal detected based on the molecular mass of the complex and the oscillation interference of the detection membrane. A baseline threshold is established to account for the complex associated to the Apta-ViBE detection membrane that does not contain the analyte. The algorithmic software treats these composite detection signals appropriately before allowing for analyte concentration to be determined.

References herein to "receptacle" include any suitable well, chamber or like entity which is capable of receiving a sample which is intended to be subjected to the method of the invention, or any combination of receptacles which may have any number of chambers. In particular, it will be appreciated that the receptacle should be designed such that the sample may be brought into fluid communication with the surface of the receptacle coated with second binding agent. It will be appreciated that the sample may be introduced to the surface of the receptacle in a static mode. Thus, in one embodiment, the receptacle additionally comprises an inlet for receiving the sample. The sample may optionally be incubated with the coated particles for a predetermined period prior to application of the magnetic field in step (c). By contrast, the sample may be introduced to the surface of the receptacle in a non-static mode. Thus, in a further embodiment, the receptacle additionally comprises an outlet for permitting the flow of sample containing the coated magnetic particles. It will therefore be appreciated that the coated magnetic particles can be contacted with the sample prior to introducing the particles into the receptacle or the sample can be introduced into the receptacle prior to or simultaneously with the introduction of the particles.

Thus, according to one embodiment of the method of the invention, the coated magnetic particles can be exposed to the sample using a variety of different formats. For example, in one embodiment, the coated magnetic particles are exposed to the sample and then introduced into the receptacle. The sample may be concentrated prior to introducing the sample-exposed particles into the receptacle. The sample may be concentrated, for example by removing the beads from the solution and resuspending in a smaller volume of liquid.

In an alternative embodiment, the sample is introduced into the receptacle prior to introducing the coated magnetic particles. In a yet further embodiment, the coated magnetic particles are introduced into the receptacle prior to adding the sample to the receptacle.

Samples suitable for use in the method of the present invention include any material suspected of containing the analyte. The sample can be used directly as obtained from the source or following one or more steps to modify the sample. In one embodiment, the sample is a biological sample. The sample can be derived from any biological source, such as a physiological fluid (e.g., blood, saliva, sputum, plasma, serum, ocular lens fluid, cerebrospinal fluid, sweat, urine, milk, ascites fluid, synovial fluid, peritoneal fluid, amniotic fluid, and the like) and fecal matter. The sample can be obtained from biological swabs such as nasal or rectal swabs. In addition, the sample can be biopsy material. The sample can be obtained from a human, primate, animal, avian or other suitable source.

The sample can be pretreated prior to use, such as preparing plasma from blood, diluting viscous fluids, and the like. The sample can also be filtered, distilled, extracted, or concentrated. For example, some small molecule drugs permeate blood cells and bind to proteins in blood (e.g. FK506). Small molecules can be extracted by adding a protein precipitation agent and cell lysing agent. For example, a mixture of zinc sulphate, polyethylene glycol and methanol can be added to blood and the solvent phase eluted after centrifugation. An alternative preparation method is to add a mixture containing protein digestion enzymes and detergents to break down the proteins and cells to release the small molecule. After centrifugation, the liquid phase is eluted and analyzed to determine the small molecule concentration in the original sample. The sample can also be treated to inactivate or modify certain activities in the sample capable of interfering with the analyte or the detection process. For example, a decomplexing antagonist can be added to the sample to disassociate the analyte from other molecules that may be bound to and/or may interfere with the ability of the capture agent to bind to the analyte.

Other liquid samples besides physiological fluids can be used, such as water, food products, and the like, for the performance of environmental or food production assays. In addition, a solid material suspected of containing the analyte can be used as the test sample. A solid test sample can be modified (e.g., homogenized, extracted, or solubilized) to form a liquid medium or to release the analyte.

The sample volume can be as little as 10 µl or as much as 250 ml. In one embodiment, the sample volume can be as little as 50 µl or as much as 5 ml. In one embodiment, the sample volume is about 1 to about 5 ml. In one embodiment, a single sample can be tested within a single receptacle. In addition, in order to test a panel of one or more analytes, a single sample can be divided into two or more aliquots. Each aliquot can be tested for a different analyte, for example, using a different receptacle for each analyte to be tested. In this way, samples can be analyzed on an individual basis, that is, as they are acquired from the patient, rather than in batch mode, e.g, accumulating multiple samples to analyze at the same time or in the same machine run.

References herein to the term "analyte" include references to, for example, the molecular structure that is recognized by the binding agent. The term analyte also includes larger molecules that contain a molecular structure that is recognized by the binding agent. The analyte can be part of a cell, for example a cell surface protein. The analyte can be an analyte of interest, chosen by the user (e.g., preselected). The analyte can be selected based on the ability to bind a binding agent of interest, for example in small molecule library screening.

As described herein, the present invention can be used to measure one or more analytes of a panel of analytes. The panel of analytes can include one or more analytes that are detected using the method described herein. The panel of analytes can include one or more analytes detected using the method described herein, in one embodiment, each analyte is detected using a separate receptacle in order to test a panel of one or more analytes, a single sample can be divided into two or more aliquots. Each aliquot can be tested for a different analyte, for example, using a different receptacle for each analyte to be tested. In this manner, panels of different analytes may be tested without requiring that multiple samples be acquired and/or that different types of apparatus be employed to test the detection of the different analytes.

In one embodiment, the analyte of interest is a small molecule. Small molecules include organic or inorganic molecules having a molecular weight of the order of about 1000 g/mol or less. Typically, small molecule analytes will contain a single or only a few binding sites. Because a small molecule has a few or only one binding site, the present invention is extremely capable of detecting and/or quantifying small molecule analytes. The small molecule can include, for example, steroids, lipids, carbohydrates, peptides, and heterocyclic compounds (e.g. bases, including co-factors such as FAD and NADH). The analyte (e.g., small molecule) can be part of a library of small organic molecules which comprise aldehydes, ketones, oximes, hydrazones, semicarbazones, carbazides, primary amines, secondary amines, tertiary amines, N-substituted hydrazines, hydrazides, alcohols, ethers, thiols, thioethers, thioesters, disulfides, carboxylic acids, esters, amides, ureas, carbamates, carbonates, ketals, thioketals, acetals, thioacetals, aryl halides, aryl sulfonates, alkyl halides, alkyl sulfonates, aromatic compounds, heterocyclic compounds, anilines, alkenes, alkynes, diols, amino alcohols, oxazolidines, oxazolines, thiazolidines, thiazolines, enamines, sulfonamides, epoxides, aziridines, isocyanates, sulfonyl chlorides, diazo compounds and/or acid chlorides, preferably aldehydes, ketones, primary amines, secondary amines, alcohols, thioesters, disulfides, carboxylic acids, acetals, anilines, diols, amino alcohols and/or epoxides, most preferably aldehydes, ketones, primary amines, secondary amines and/or disulfides and combinations thereof.

The analyte of interest can also be a biological analyte, such as a polypeptide, a nucleic acid, a carbohydrate, a nucleoprotein, a glycopeptide or a glycolipid. Useful analytes include, for example, enzymes, steroids, hormones, transcription factors, growth factors, immunoglobulins, steroid receptors, nuclear proteins, signal transduction components, allosteric enzyme regulators, and the like. Analytes of interest can be obtained, for example, commercially, recombinantly, synthetically, or by purification from a natural source. In particular embodiments, the analyte of interest is associated with a specific human disease or condition. Suitable growth factors include, for cytokines such as erythropoietin/EPO, granulocyte colony stimulating receptor, granulocyte macrophage colony stimulating receptor, thrombopoietin (TPO), IL-2, IL-3, IL-4, IL-5, IL-6, IL-10, EL-11, DL-12, growth hormone, prolactin, human placental lactogen (LPL), CNTF, and octostatin. Suitable steroids include, but are not limited to, estradiol, progesterone, testosterone, and derivatives thereof. Hormone analytes, of relevance to prenatal testing, include alpha-fetoprotein (AFP), human chorionic gonadotrophin (hCG), estriol and inhibin. Other suitable analytes include, for example, insulin, insulin-like growth factor 1 (IGF-1), epidermal growth factor (EGF), vascular endothelial growth factor (VEGF), placental growth factor (PLGF),TGF-α and TGF-β), other hormones and receptors such as bone morphogenic factors, folical stimulating hormone (FSH), and leutinizing hormone (LH), tissue necrosis factor (TNF), apoptosis factor-1 and -2 (AP-1 and AP-2), and mdm2. A biological analyte also includes cellular analytes that include, for example, a suitable detectable marker, such as a surface receptor.

The analyte of interest can also be a therapeutic drug where it would be useful to measure the levels of the drug in a patient sample, for example for drug management purposes.

The analyte of interest can be a pathogen or microbe, such as bacteria or bacterial spores, viruses, parasites, prions or other pathogens (such as Giardia, malaria and cryptosporidia) or their cell wall or surface components such as gram-positive peptidoglycans, lipoteichoic and teichoicacids, and gram- negative endotoxin (e.g.) lipopolysaccharide), or other toxins released by the organism, such as, for example, staphylococcal alpha toxin, beta toxin, protein A; clostridium difficile toxins A and B; shiga toxin; and botulinum toxin.

The analytes can be, for example, the same molecular species of interest. In an alternative embodiment, the different analytes are part of a larger molecule. Therefore, the analyte can be a particular binding site attached to or contained within a larger molecule. As such, the different analytes being detected can be part of different molecules.

According to a second aspect of the invention there is provided a method of diagnosing or monitoring a disease within a subject, wherein said diagnostic method comprises the steps of:
(a) preparing one or more magnetic complexes comprising a magnetic particle coated with a first binding agent capable of specific binding to an analyte biomarker which is characteristic for the disease, wherein a spacer element is present between the first binding agent and the magnetic particle;
(b) adding the one or more magnetic complexes prepared in step (a) to a biological sample obtained from the subject within a receptacle, wherein a surface of a receptacle is coated with a second binding agent capable of specific binding to the analyte biomarker which is characteristic for the disease, such that at least one of said first and second binding agents is a DNA or RNA oligonucleotide;
(c) temporarily applying a magnetic field to the surface of the receptacle which is coated with the second binding agent; and
(d) detecting the presence of magnetic complexes which remain bound to the surface of the receptacle following step (c) which is indicative of the presence of the analyte biomarker characteristic for the disease within the biological sample, or a binding value which is above or below a threshold value characteristic for the disease may be indicative of presence of the disease or risk for the disease.

Monitoring methods of the invention can be used to monitor onset, progression, stabilisation, amelioration and/or remission of the disease.

In methods of diagnosing or monitoring according to the invention, detecting and/or quantifying the analyte biomarker in a biological sample from a test subject may be performed on two or more occasions. Comparisons may be made between the level of analyte biomarker in samples taken on two or more occasions. Assessment of any change in the level of the analyte biomarker in samples taken on two or more occasions may be performed. Modulation of the analyte biomarker level is useful as an indicator of the state of the disease or predisposition thereto. An increase in the level of the biomarker, over time may be indicative of onset or progression, i.e. worsening of this disease, whereas a decrease in the level of the analyte biomarker may indicate amelioration or remission of the disease, or vice versa.

A method of diagnosis or monitoring according to the invention may comprise quantifying the analyte biomarker in a test biological sample from a test subject and comparing the level of the analyte present in said test sample with one or more controls.

The control used in a method of the invention can be one or more control(s) selected from the group consisting of: the level of biomarker analyte found in a normal control sample from a normal subject, a normal biomarker analyte level; a normal biomarker analyte range, the level in a sample from a subject with the disease, or a diagnosed predisposition thereto.

The term "diagnosis" as used herein encompasses identification, confirmation, and/or characterisation of the disease, or predisposition thereto. By predisposition it is meant that a subject does not currently present with the disease, but is liable to be affected by the disease in time. Methods of monitoring and of diagnosis according to the invention are useful to confirm the existence of a disease, or predisposition thereto; to monitor development of the disease by assessing onset and progression, or to assess amelioration or regression of the disease. Methods of monitoring and of diagnosis are also useful in methods for assessment of clinical screening, prognosis, choice of therapy, evaluation of therapeutic benefit, i.e. for drug screening and drug development.

Efficient diagnosis and monitoring methods provide very powerful "patient solutions" with the potential for improved prognosis, by establishing the correct diagnosis, allowing rapid identification of the most appropriate treatment (thus lessening unnecessary exposure to harmful drug side effects), reducing relapse rates.

Methods for monitoring efficacy of a therapy can be used to monitor the therapeutic effectiveness of existing therapies and new therapies in human subjects and in non-human animals (e.g. in animal models). These monitoring methods can be incorporated into screens for new drug substances and combinations of substances.

Suitably, the time elapsed between taking samples from a subject undergoing diagnosis or monitoring may be continuous, or at intervals such as 3 days, 5 days, a week, two weeks, a month, 2 months, 3 months, 6 or 12 months. Samples may be taken prior to and/or during and/or following therapy. Samples can be taken at intervals over the remaining life, or a part thereof, of a subject.

It will be appreciated that the method of the invention may be used to detect the presence of bacteria, i.e. diagnosis of infection by a specific bacterial species, such as Staphylococcus sp. such as *Staphylococcus aureus* (especially methicilin resistant *Staphylococcus aureus* (MRSA)), Clostridia sp (e.g. *Clostridium difficile, Clostridium tetani* and *Clostridium botulinum*), Enterobacter species, *Mycobacterium tuberculosis*, Shigella sp. such as *Shigella dysenteriae*, Campylobacter sp. such as *Campylobacter jejuni*, Enterococcus sp. such as *Enterococcus faecalis, Bacillus anthracis, Yersinia pestis, Bordetella pertussis,* Streptococcal species, *Salmonella thyphimurim, Salmonella enterica*, Chlamydia species, *Treponema pallidum, Neisseria gonorrhoeae, Borrelia burgdorferi, Vibrio cholerae, Corynebacterium diphtheriae, Helicobacter pylori,* Gram-negative multidrug resistant (MDR)-pathogens such as *Acinetobacter baumannii, Pseudomonas aeruginosa, Klebsiella pneumoniae*, and *Escherichia coli*. Thus, according to a further aspect of the invention, there is provided a method of diagnosing infection by a specific bacterial species, which comprises the method as hereinbefore defined. In one embodiment of this aspect of the invention, the analyte comprises a bacterial target. It will be appreciated that the binding agents in this aspect of the invention could be designed to peptide (or other surface molecule) targets which are well-conserved, and consistent across bacterial species. In another embodiment a binding agent is selected to be narrow and specific to a certain subtype of the bacterial species, and is not consistent between polyclonal varieties, such as a binding agent specifically directed to a peptide (or other target) present on MRSA but not wild type *S*. *aureus*. Aptamers have unique advantages over other potential binding agents to be used for this purpose because (i) they come from such a large initial pool of possible combinations, that a suitable one can almost always be found - even for targets for which there is no extant antibody or other binding agent; and (ii) they are highly specific and therefore well-suited for diagnostic purposes for which a low number of false-positives is desirable.

In one embodiment, the analyte of interest is *Staphylococcus aureus.* It will be appreciated that when the analyte of interest is *Staphylococcus aureus* it is possible to design aptamers intended to recognize and bind specific peptides characteristic for such a bacteria. Thus, in one embodiment, the analyte of interest is sortase B of *Staphylococcus aureus.* Aptamers which may be suitable for sortase B of *Staphylococcus aureus* include the following thioaptamers (wherein the underlined T nucleotide refers to the thiolated nucleotide):

In an alternative embodiment, the analyte of interest is Protein A of *Staphylococcus aureus.* Aptamers which may be suitable for Protein A of *Staphylococcus aureus* include the following thioaptamers (wherein the underlined T nucleotide refers to the thiolated nucleotide):

Alternatively, suitable aptamers for the detection of whole cell *Staphylococcus aureus* include aptamers that demonstrated high specificity and affinity to *Staphylococcus aureus* individually. The five aptamers recognizing different molecular targets by a process of competitive experiment, as described in Cao X, et al. 2009. Combining use of a panel of ssDNA aptamers in the detection of Staphylococcus aureus. Nucleic Acids Research. Vol. 37. No. 14. PP 4621-4628.

In one embodiment, the analyte of interest is *Clostridium difficile.* It will be appreciated that when the analyte of interest is *Clostridium difficile* it is possible to design aptamers intended to recognize and bind specific peptides characteristic for such a bacteria. Thus, in one embodiment, the analyte of interest is toxin A of *Clostridium difficile.* Aptamers which may be suitable for toxin A of *Clostridium difficile* include:
CDA-19 - 5'-ATT CTA TGA CTA CCT ACA CCT CAC TGT CTC TCC ACC TTT TTG-3' (SEQ ID NO: 22)
CDA-21 - 5'-CAC CCG ACC GAA CTT CTC TTT TTC CTA CAT ATA GCA TCG TAA-3'(SEQ ID NO: 23)
CDA-23 - 5'-ACC CAC GAA TTT CCA CAC ACT ATC CTA TCT CCA CTA ATC TA-3'(SEQ ID NO: 24)
CDA-29 - 5'-CCC TAA CAA ACA TGT CAA TTC AGA GAT TTT TAC CTA ACA TGC-3'(SEQ ID NO: 25)
CDA-33 - 5'-GAA CAT TAA CAC TCG CCG GAA TAT TCC AAC TAC CTT TTA CCT-3'(SEQ ID NO: 26)
CDA-34 - 5'-GTT TGG ATT GTA CCA CCA TTC AAT TAA CTT ACT ATA CTA TTA-3'(SEQ ID NO: 27)
CDA-37 - 5'-TCC CAT ACC ACA TTC CCT TCT CAA ACT ATC AAA AGC TCA GGG-3'(SEQ ID NO: 28)
CDA-53 - 5'-CAC CAC ACT TTT CCT CAT TCA ACT AAC GTT CGT CAC CGC AAT-3'(SEQ ID NO: 29)

In an alternative embodiment, the analyte of interest is toxin B of *Clostridium difficile*. Aptamers which may be suitable for toxin B of *Clostridium difficile* include:
CDB-4 - 5'-TAA CAA TTT CTT TTA CTT CCA TTT CCT TAT GCA CTA AAT CTC-3'(SEQ ID NO: 30)
CDB-5 - 5'-AAC ACT CTT TTC TTT ATT TAT TGT CTC TTT TAC TTT TTT TT-3'(SEQ ID NO: 31)
CDB-12 - 5'-TAC ACC TTT TTT TAA TCC CTT ACA TTT ACC ATT CTT TAT CA-3'(SEQ ID NO: 32)
CDB-34 - 5'-ACT TTC CTT ATT ATC TTT CCC TAT TTT TTG TTT CTA TTC TAT-3'(SEQ ID NO: 33)
CDB-46 - 5'-TTT TAC TCA TCC CAA TTA CCT CTT TTC TAA TAA CTC GCC CTT-3'(SEQ ID NO: 34)

It will also be appreciated that the detection method of the invention may be used to detect the presence of a virus, i.e. diagnosis of infection by a specific viral species, such as Influenza viruses (such as Influenza A and Influenza B), CMV, EBV, Human Immunodeficiency virus (HIV), Hepatitis viruses (such as Hepatitis B virus (HBV) and Hepatitis C virus (HCV)), Rous sarcoma virus (RSV), Herpes viruses (such as Herpes Simplex Virus (HSV) and Herpes Zoster Virus (HZV)), Rhinovirus, Yellow Fever, Group B Coxsachieviruses, (CB1, CB2, CB3, CB4, CB5, and CB6), Canine Parvovirus (CPV), Vaccina Virus, T4-like virus, Adenovirus, Avian flu, rhinovirus, coronavirus (e.g., SARS), West Nile Virus, Cytomegalovirus, Epstein-Barr virus and Ebola virus. Thus, according to a further aspect of the invention, there is provided a method of diagnosing infection by a specific viral species, which comprises the method as hereinbefore defined. In one embodiment of this aspect of the invention, the analyte comprises a viral target. It will be appreciated that the binding agents in this aspect of the invention could be designed to peptide or protein (or other surface molecule) targets which are well-conserved, and may be consistent across species. In another embodiment a binding agent is selected which is known to be narrow and specific to a certain subtype of the species, and is not consistent between polyclonal varieties, such as a binding agent specifically directed to a peptide (or other target) present on a certain virion, or cell infected with a certain virion, but not another strain of the same virus. Another advantage of using an aptamer as the binding agent in this context is the rapid and programmable-discovery nature of aptamers, therefore new specific diagnostics could be developed very rapidly for new targets, for new pandemic-causing strains etc.

In one embodiment, the analyte of interest is Influenza. It will be appreciated that when the analyte of interest is Influenza it is possible to design aptamers intended to recognize and bind specific peptides characteristic for such a virus. Thus, in one embodiment, the analyte of interest is the M2 coat protein of Influenza. Aptamers which may be suitable for the M2 coat protein of Influenza include the following thioaptamers to the M2e peptide (wherein the underlined T nucleotide refers to the thiolated nucleotide):

Optionally, suitable aptamers for the detection of influenza include aptamers designed to complement receptor-binding region of the influenza hemagglutinin molecule, as described in Jeon HS, Kayhan, B, Ben-Yedidia T and Arnon, R. 2004. A DNA Aptamer Prevents Influenza Infection by Blocking the Receptor Binding Region of the Viral Hemagglutinin. The Journal of Biological Chemistry. Vol. 279, No. 46. PP. 48410-48419.

When the analyte is a viral target, in one embodiment, the first and/or second binding agents may comprise any of the monoclonal antibodies listed below in Table 1:

**Table 1: Examples of Monoclonal Antibodies as First and/or Second Binding Agents for use in the detection of a virus**

| ***Name*** | ***Trade Name*** | ***Type*** | ***Source*** | ***Target*** | ***Use*** |
|---|---|---|---|---|---|
| Exbivirumab | | mab | human | hepatitis B surface antigen | hepatitis B |
| Felvizumab | | mab | humanized | respiratory syncytial virus | respiratory syncytial virus infection |
| Foravirumab | | mab | human | rabies virus glycoprotein | rabies (prophylaxis) |
| Ibalizumab | | mab | humanized | CD4 | HIV infection |
| Libivirumab | | mab | human | hepatitis B surface antiqen | hepatitis B |
| Motavizumab | Numax | mab | humanized | respiratory syncytial virus | respiratory syncytial virus (prevention) |
| Palivizumab | Synagsis, Abbosynagis | mab | humanized | respiratory syncytial virus | respiratory syncytial virus (prevention) |
| PRO 140 | | | humanized | CCR5 | HIV infection |
| Rafivirumab | | mab | human | rabies virus glycoprotein | rabies (prophylaxis) |
| Reqavirumab | | mab | human | cytomegalovirus glycoprotein B | cytomegalovirus infection |
| Sevirumab | | | human | cytomegalovirus | cytomegalovirus infection |
| Tuvirumab | | | human | hepatitis B virus | chronic hepatitis B |

The detection method of the invention also finds particular utility in the diagnosis of infection with a particular species of pathogen (i.e bacteria or virus) which makes use of detecting T cells. Memory T cells are a subset of antigen-specific T cells that persist long-term after an individual has recovered from an infection. They quickly expand to large numbers of effector T cells upon re-exposure to their cognate antigen. Effector T cells migrate to sites of infection to eradicate the pathogen by releasing a cytokine response (Interferon Gamma being the most common one). These cells survive *in vivo* for a shorter time than memory T cells and are therefore a good indicator of the presence of a specific pathogen in an individual within an immediate timeframe (the usual life span of an effector T cell is around four weeks). In this embodiment, the effector T cells present in a blood sample could be incubated with a series of antigens of interest separately (i.e. a pathogen) in order to develop a specific and immediate response against the target antigen (this is mimicking the presence of a pathogen *in vivo*). The method of the invention could then be conducted such that a target analyte could be identified for given populations of T cells, and these T cells could be identified and counted, which are specific for the presence of a specific antigen. By taking a sample infected with a particular antigen, the method of the invention could then rapidly be used to diagnose whether said individual is infected with a given pathogen. Thus, according to a further aspect of the invention, there is provided a method of diagnosing infection by a specific bacterial species, which comprises the method as hereinbefore defined which targets specific antigens on effector T cells.

It will also be appreciated that the detection method of the invention may be used to detect the presence of bacteria, fungi, viruses and any other microorganisms, such as Mycobacterium tuberculosis (bacterium) Aspergillus (fungus) epstein barr virus (EBV), cytomegalovirus (CMV), etc. Thereby providing a diagnosis of a bacterial infection, fungal infection, viral infection or any other pathogen based infection. Thus, according to a further aspect of the invention, there is provided a method of diagnosing a pathogenic infection, which comprises the method as hereinbefore defined. In one embodiment of this aspect of the invention, the pathogenic infection comprises a disease such as Aspergillosis which is a group of diseases which can result from Aspergillus infection and includes invasive Aspergillosis, ABPA, CPA and Aspergilloma.

It will also be appreciated that the detection method of the invention may be used to detect the presence of cancer, i.e. diagnosis of cancer. Thus, according to a further aspect of the invention, there is provided a method of diagnosing cancer, which comprises the method as hereinbefore defined. In one embodiment of this aspect of the invention, the analyte comprises a tumour antigen or other molecule associated with a tumour, including those whose presence being lower than normal would indicate the presence of a tumour. It will be appreciated that the binding agents in this aspect of the invention could be designed to bind certain tumour antigens known to exist as a result of malignant cells, and may be present in tissue samples either free-floating or bound to malignant cells. In one embodiment, the analyte for cancer is E-selectin. E-selectin, also known as CD62 antigen-like family member E (CD62E), endothelial-leukocyte adhesion molecule 1 (ELAM-1), or leukocyte-endothelial cell adhesion molecule 2 (LECAM2), is a cell adhesion molecule expressed only on endothelial cells activated by cytokines. E-selectin is an emerging biomarker for the metastatic potential of some cancers including colorectal cancer, pancreatic cancer and breast cancer.

In an alternative embodiment, the analyte for cancer is CD4. CD4 (cluster of differentiation 4) is a glycoprotein expressed on the surface of T helper cells, regulatory T cells, monocytes, macrophages, and dendritic cells. It has recently been shown that levels of CD4 cells are linked with cancer (Guiguet M et al. The Lancet Oncology (online edition), 2009).

When the analyte is a tumour antigen, in one embodiment, the first and/or second binding agents specific for the tumour antigen is labyrinthin. Labyrinthin is known to be a marker for cancer (US Patent No. 7,329,743) and was previously known as the mouse monoclonal antibody (MCA) 44-3A6 (Radosevich et al (1985) Cancer Research 45, 5808-5812).

When the analyte is a tumour antigen, in one embodiment, the first and/or second binding agents may comprise any of the monoclonal antibodies listed below in Table 2:

**Table 2: Examples of Monoclonal Antibodies as First and/or Second Binding Agents for use in the diagnosis of cancer**

| ***Name*** | ***Trade Name*** | ***Type*** | ***Source*** | ***Target*** | ***Use*** |
|---|---|---|---|---|---|
| 3F8 | | | mouse | GD2 | neuroblastoma |
| Abaqovomab | | mab | mouse | CA-125 | ovarian |
| Adecatumumab | | mab | human | EpCAM | prostate/breast |
| Afutuzumab | | mab | humanized | CD20 | lymphoma |
| Alacizumab pegol | | F(ab')2 | humanized | VEGFR2 | cancer |
| Alemtuzumab | Campath, MacCampath | mab | humanized | CD52 | CLL, CTCL |
| Altumomab pentetate | Hybri-ceaker | mab | mouse | CEA | colorectal-diagnosis |
| Anatumomab mafenatox | | Fab | mouse | TAG-72 | non-small cell lung carcinoma |
| Apolizumab | | mab | humanized | HLA-DR | hematological |
| Arcitumomab | CEA-Scan | Fab' | mouse | CEA | gastrointestinal |
| Bavituximab | | mab | chimeric | Phosphatidylserine | cancer |
| Bectumomab | LymphoScan | Fab' | mouse | CD22 | non-Hodgkin's lymphoma |
| Belimumab | Benlysta, LymphoStat-B | mab | human | BAFF | non-Hodgkin lymphoma etc. |
| Bevacizumab | Avastin | mab | humanized | VEGF-A | metastatic |
| Bivatuzumab mertansine | | mab | humanized | CD44 v6 | squamous cell carcinoma |
| Blinatumomab | | BiTE | mouse | CD19 | cancer |
| Brentuximab vedotin | | - mab | chimeric | CD30 | hematologic cancers |
| Cantuzumab mertansine | | mab | humanized | mucin CanAg | colorectal |
| Capromab pendetide | Prostascint | mab | mouse | prostatic carcinoma cells | prostate-detection |
| Catumaxomab | Removab | 3funct | rat/mouse | EpCAM, CD3 | ovarian, gastric |
| CC49 | | mab | mouse | TAG-72 | tumor detection |
| Cetuximab | Erbitux | mab | chimeric | EGFR | colorectal/head/neck |
| Citatuzumab bogatox | | Fab | humanized | EpCAM | ovarian/solid tumors |
| Cixutumumab | | mab | human | IGF-1 receptor | solid tumors |
| Clivatuzumab tetraxetan | | mab | humanized | MUC1 | pancreatic |
| Conatumumab | | mab | human | TRAIL-R2 | cancer |
| Dacetuzumab | | mab | humanized | CD40 | hematologic cancers |
| Denosumab | Prolia | mab | human | RANKL | bone metastases etc |
| Detumomab | | mab | mouse | B-lymphoma | lymphoma |
| Ecromeximab | | mab | chimeric | GD3 ganglioside | malignant melanoma |
| Edrecolomab | Panorex | mab | mouse | EpCAM | colorectal carcinoma |
| Elotuzumab | | mab | humanized | SLAMF7 | multiple myeloma |
| Epratuzumab | | mab | humanized | CD22 | cancer |
| Ertumaxomab | Rexomun | 3funct | rat/mouse | HER2/neu , CD3 | breast cancer etc. |
| Etaracizumab | Abegrin | mab | humanized | integrin αvβ3 | melanoma, prostate, ovarian etc. |
| Farletuzumab | | mab | humanized | folate receptor 1 | ovarian |
| Figitumumab | | mab | human | IGF-1 receptor | adrenocortical carcinoma, non-small cell lung carcinoma etc. |
| Fresolimumab | | mab | human | TGT-β | cancer |
| Galiximab | | mab | chimeric | CD80 | B-cell lymphoma |
| Gemtuzumab ozogamicin | Mylotarg | mab | humanized | CD33 | acute myelogenous leukemia |
| Glembatumumab vedotin | | mab | human | GPNMB | melanoma, breast |
| Ibritumomab tiuxetan | Zevalin | mab | mouse | CD20 | non-Hodgkin's lymphoma |
| Igovomab | Indimacis-125 | F(ab')2 | mouse | CA-125 | ovarian-diagnosis |
| Intetumumab | | mab | human | CD51 | solid tumors |
| Inotuzumab ozogamicin | | mab | humanized | CD22 | cancer |
| Ipilimumab | | mab | human | CD152 | melanoma |
| Labetuzumab | CEA-Cide | mab | humanized | CEA | colorectal |
| Lexatumumab | | mab | human | TRAIL-R2 | cancer |
| Lintuzumab | | mab | humanized | CD33 | cancer |
| Lucatumumab | | mab | human | CD40 | multiple myeloma, non-Hodgkin's lymphoma, Hodgkin's lymphoma |
| Lumiliximab | | mab | chimeric | CD23 | chronic lymphocytic leukemia |
| Mapatumumab | | mab | human | TRAIL-R1 | cancer |
| Matuzumab | | mab | humanized | EGFR | colorectal, lung and stomach cancer |
| Milatuzumab | | mab | humanized | CD47 | multiple myeloma, other hematological malignancies |
| Mitumomab | | mab | mouse | GD3 ganglioside | small cell lung carcinoma |
| Nacolomab tafenatox | | Fab | mouse | C242 antigen | colorectal |
| Naptumomab estafenatox | | Fab | mouse | 5T4 | non-small cell lung/renal cell carcinoma |
| Necitumumab | | mab | human | EGFR | non-small cell lung carcinoma |
| Nimotuzumab | Theracim, Theraloc | mab | humanized | EGFR | squamous cell carcinoma, head and neck cancer, nasopharyngeal cancer, glioma |
| Nofetumomab merpentan | Verluma | Fab | mouse | | cancer-diagnosis |
| Ofatumumab | Arzerra | mab | human | CD20 | chronic lymphocytic leukemia etc. |
| Olaratumab | | mab | human | PDGF-R α | cancer |
| Oportuzumab monatox | | scFv | humanized | EPCAM | cancer |
| Oregovomab | OvaRex | mab | mouse | CA-125 | ovarian |
| Panitumumab | Vectibix | mab | human | EGFR | colorectal |
| Pemtumomab | Theragyn | | mouse | MUC1 | cancer |
| Pertuzumab | Omnitarg | mab | humanized | HER2/neu | cancer |
| Pintumomab | | mab | mouse | adenocarc inoma antigen | adenocarcinom a-imaging |
| Pritumumab | | mab | human | vimentin | brain |
| Ramucirumab | | mab | human | VEGFR2 | solid tumors |
| Rilotumumab | | mab | human | HGF | solid tumors |
| Rituximab | MabThera, Rituxan | mab | chimeric | CD20 | lymphomas, leukemias |
| Robatumumab | | mab | human | IGF-1 receptor | cancer |
| Satumomab pendetide | | mab | mouse | TAG-72 | cancer-diagnosis |
| Sibrotuzumab | | mab | humanized | FAP | cancer |
| Tacatuzumab tetraxetan | AFP-Cide | mab | humanized | alpha-fetoprotein | cancer |
| Taplitumomab paptox | | mab | mouse | CD19 | cancer |
| Tenatumomab | | mab | mouse | tenascin C | cancer |
| TGN1412 | | | humanized | CD28 | chronic lymphocytic leukemia |
| Ticilimumab | | mab | human | CTLA-4 | cancer |
| Tigatuzumab | | mab | humanized | TRAIL-R2 | cancer |
| TNX-650 | | | humanized | IL-13 | Hodgkin's lymphoma |
| Tositumomab | Bexxar | | mouse | CD20 | follicular lymphoma |
| Trastuzumab | Herceptin | mab | humanized | HER2/neu | breast |
| Tremelimumab | | mab | human | CTLA-4 | cancer |
| Tucotuzumab celmoleukin | | mab | humanized | EpCAM | cancer |
| Veltuzumab | | mab | humanized | CD20 | non-Hodgkin's lymphoma |
| Volociximab | | mab | chimeric | integrin α5β1 | solid tumors |
| Votumumab | HumaSPECT | mab | human | tumor antigen CTAA16.8 8 | colorectal tumors |
| Zalutumumab | HuMax-EGFr | mab | human | EGFR | squamous cell carcinoma of the head and neck |
| Zanolimumab | HuMax-CD4 | mab | human | CD4 | T-cell lymphoma |

It will also be appreciated that the detection method of the invention may be used to detect the presence of an autoimmune disorder, i.e. diagnosis of an autoimmune disorder. Thus, according to a further aspect of the invention, there is provided a method of diagnosing an autoimmune disorder, which comprises the method as hereinbefore defined. In one embodiment of this aspect of the invention, the analyte comprises an autoantibody, or other automimmune biomarker, such as the Fc portion of an autoantibody. It will be appreciated that the binding agents in this aspect of the invention could be designed to bind the Fc portion of a particular autoantibody. In an alternative embodiment, one of the first or second binding agents may be used which specifically binds the Fc portion of a particular autoantibody and the other binding agent may be used which specifically binds to the antigen of the autoantibody. This embodiment provides the advantage of effective diagnosis in the event that the autoantibody being sought as the analyte is pre-bound in serum to its antigen (e.g. RF-IgG) and cannot easily be removed. Alternatively, the pre-bound autoantibodies (i.e. RF bound to IgG in serum) may be separated prior to conducting the diagnostic method of this aspect of the invention.

Examples of autoantibodies include PBC: Anti-gp210, Anti-p62, Anti-sp100; ENA: Anti-topoisomerase/ScI-70, Anti-Jo1, ENA4 (Anti-Sm, Anti-nRNP, Anti-Ro, Anti-La); Anti-centromere; Anti-dsDNA; Anti-mitochondrial antibody; Anti-cardiolipin; Anti-cytoplasm antibody; Anti-neutrophil cytoplasmic; Anti-actin/smooth muscle; Antimicrosomal antibody;Anti-ganglioside; Anti-GBM; Anti-thrombin; Lupus anticoagulant; Anti-transglutaminase; RA (Rheumatoid factor/anti-IgG, Anti-citrullinated peptide); Anti-phospholipid; and Anti-apolipoprotein.

It will also be appreciated that the detection method of the invention may be used to detect the presence of a cardiovascular disorder, i.e. diagnosis of a cardiovascular disorder. Thus, according to a further aspect of the invention, there is provided a method of diagnosing a cardiovascular disorder, which comprises the method as hereinbefore defined. In one embodiment of this aspect of the invention, the analyte comprises a biomarker indicative of a cardiovascular disorder, such as: Troponin T; YKL-40; activated endothelial progenitor cells (EPCs); carbamylated biomarkers such as carbamylated albumin, carbamylated fibrinogen, carbamylated immunoglobulin and carbamylated apolipoprotein A; Tumor Necrosis Factor Alpha Precursor; Lysosomal-associated Membrane Protein; Interleukin-5 Precursor; Interleukin-6 Precursor; C-C Motif Chemokine Precursor; C-C Motif Chemokine Precursor RANTES; Cathepsin L1 Precursor; Adenylate Kinase; Leukotriene B4 Receptor; Complement Factor D; Secreted Phosphoprotein; Small Inducible Cytokine A17 Precursor; C-X-C Motif Chemokine Precursor; Tumor Necrosis Factor Ligand Superfamily Member (RANKL); C-C Motif Chemokine Precursor; 72 kDa Type IVCollagenase Precursor; Neutrophil Collagenase Precursor; fatty acid binding protein; calpain, (m/II) large subunit; Macrophage Migration Inhibitory Factor; Cathepsin S Precursor; Interleukin Precursor; soluble ICAM-1; Galectin-3 - binding protein; NPR3; CTGF, PRSS23; CRTAC; FN1; LTBP2; TGFB2; ST2/Interleukin 1 Receptor Like 1 (IL1RL1); Interleukin 33 (IL-33); a natriuretic peptide, e.g., brain natriuretic peptide (BNP); prohormone BNP (proBNP); N-Terminal proBNP (NT-proBNP); atrial natriuretic peptide (ANP); proANP; NT-proANP; S100A12; ApoC-IV; Paraoxonase 1; C3; C4; ApoA-IV; ApoE; ApoL1; C4B1; Histone H2A; ApoC-II; ApoM; Vitronectin; Haptoglobin-related protein; and Clusterin. In one embodiment, the cardiovascular disorder is selected from acute coronary syndrome, acute myocardial infarction (AMI/ STEMI/ST-elevation), myocardial infarction (heart attack), unstable angina pectoris/UAP/non-ST-elevation myocardial infarction, aneurysms, angina, atherosclerosis, coronary artery disease (CAD), ischemic heart disease, ischemic myocardium, arrhythmia, atrial fibrillation, cardiac arrhythmia, ventricular tachycardia, ventricular fibrillation, cardiac and sudden cardiac death, cardiomyopathy, congestive heart failure, heart failure, diastolic and systolic ventricular dysfunction, dilated cardiomyopathy, high blood pressure (hypertension), hypertrophic cardiomyopathy, valve disease, mitral valve prolapse, mitral valve regurgitation and/or stenosis, aortic valve regurgitation and/or stenosis, myocarditis and venous thromboembolism.

When the method of the invention is used in the diagnosis of a cardiovascular disorder the first and/or second binding agents may comprise any of the monoclonal antibodies listed below in Table 3:

**Table 3: Examples of Monoclonal Antibodies as First and/or Second Binding Agents for use in the diagnosis of a cardiovascular disorder**

| ***Name*** | ***Trade Name*** | ***Type*** | ***Source*** | ***Target*** | ***Use*** |
|---|---|---|---|---|---|
| Abciximab | | mab | chimeric | CD41(integrin-alpha-IIb) | platelet aggregation inhibitor |
| Erlizumab | | F(ab') 2 | humanized | ITGB2 | heart attack |
| Imciromab | Myoscint | mab | mouse | cardiac myosin | cardiac imaging |
| Pexelizumab | | scFv | humanized | C5 | reduction of side effects of cardiac surgery |
| Tadocizumab | | Fab | humanized | integrin αIIbβ3 | percutaneous coronary intervention |

It will also be appreciated that the detection method of the invention may be used to detect the presence of a degenerative disorder, such as a neurodegenerative disorder, i.e. diagnosis of a degenerative disorder, such as a neurodegenerative disorder. Thus, according to a further aspect of the invention, there is provided a method of diagnosing a degenerative disorder, such as a neurodegenerative disorder, which comprises the method as hereinbefore defined. In one embodiment of this aspect of the invention, the analyte comprises a biomarker indicative of a degenerative disorder, such as a neurodegenerative disorder, such as: certain Tau proteins; GCSF; IFN-g; IGFBP-1; BMP-6; BMP-4; Eotaxin-2; IGFBP- 2; TARC; RANTES; ANG; PARC; Acrp30; AgRP(ART); TIMP-1; TIMP-2; ICAM-1; TRAIL R3; uPAR; IGFBP-4; Leptin (OB); PDGF-BB; EGF; BDNF; NT-3; NAP-2; IL-1ra; MSP-a; SCF; TGF-b3; TNF-b; MIP-1d; IL-3; FGF-6; IL-6 R; sTNF RII; AXL; bFGF; FGF-4; CNTF; MCP-1; MIP-1b; TPO; VEGF-B; IL-8; FAS; EGF-R; basic fibroblast growth factor (bFGF); Beta-amyloid and BB homodimeric platelet derived growth factor (PDGF-BB). In one embodiment, the neurodegenerative disorder is selected from Alzheimer's disease, Parkinson's disease, Huntington's disease, Mild Cognitive Impairment (MCI), Multiple Sclerosis, Bovine spongiform encephalopathy and amyotrophic lateral sclerosis (ALS).

When the method of the invention is used in the diagnosis of multiple sclerosis the first and/or second binding agents may comprise any of the monoclonal antibodies listed below in Table 4:

**Table 4: Examples of Monoclonal Antibodies as First and/or Second Binding Agents for use in the diagnosis of multiple sclerosis**

| ***Name*** | ***Trade Name*** | ***Type*** | ***Source*** | ***Target*** | ***Use*** |
|---|---|---|---|---|---|
| Briakinumab | | mab | human | IL-12, IL-23 | multiple sclerosis |
| Natalizumab | Tysabri | mab | humanized | integrin α4 | multiple sclerosis |
| Priliximab | | mab | chimeric | CD4 | multiple sclerosis |
| Ustekinumab | Stelara | mab | human | IL-12, IL-23 | multiple sclerosis |

It will also be appreciated that the detection method of the invention may be used to detect the presence of a psychiatric disorder, i.e. diagnosis of a psychiatric disorder. Thus, according to a further aspect of the invention, there is provided a method of diagnosing a psychiatric disorder which comprises the method as hereinbefore defined. In one embodiment of this aspect of the invention, the analyte comprises a biomarker indicative of a psychiatric disorder, such as a neurodegenerative disorder, such as those described in WO 2009/077763, WO 2008/125805, WO 2008/107701, WO 2008/107700, WO 2008/107699, WO 2008/090319, WO 2008/020192, WO 2007/083146 and WO 2006/085122. In one embodiment, the psychiatric disorder is selected from Acute Stress Disorder, Adjustment Disorder, Adjustment Disorder with Anxiety, Adjustment Disorder with Depressed Mood, Adjustment Disorder with Disturbance of Conduct, Adjustment Disorder with Mixed Anxiety and Depressed Mood, Adjustment Disorder with Mixed Disturbance of Emotions and Conduct, Agoraphobia without History of Panic Disorder Anxiety Disorders, Anorexia Nervosa Eating Disorders, Antisocial Personality Disorder, Personality Disorders, Anxiety Disorder Due to Medical Condition, Anxiety Disorder NOS, Avoidant Personality Disorder, Bipolar Disorder NOS, Bipolar I Disorder Most Recent Episode Depressed (in full remission), Bipolar I Disorder Most Recent Episode Depressed (in partial remission), Bipolar I Disorder most recent episode depressed (mild), Bipolar I Disorder Most Recent Episode Depressed (Moderate), Bipolar I Disorder most recent episode depressed (severe with psychotic features), Bipolar I Disorder, most recent episode depressed (severe without psychotic features), Bipolar I Disorder most recent episode depressed (unspecified), Bipolar I Disorder most recent episode manic (in full remission), Bipolar I Disorder most recent episode manic (in partial remission), Bipolar I Disorder most recent episode manic (mild), Bipolar I Disorder most recent episode manic (moderate), Bipolar I Disorder most recent episode manic (severe with psychotic features), Bipolar I Disorder most recent episode manic (severe without psychotic features), Bipolar I Disorder most recent episode manic (unspecified), Bipolar I Disorder most recent episode mixed (in full remission), Bipolar I Disorder, most recent episode mixed in partial remission, Bipolar I Disorder most recent episode mixed (mild), Bipolar I Disorder most recent episode mixed (moderate), Bipolar I Disorder most recent episode mixed (severe with psychotic features), Bipolar I Disorder most recent episode mixed severe without psychotic features, Bipolar I Disorder most recent episode mixed (unspecified), Bipolar I Disorder most recent episode unspecified, Bipolar I Disorder most recent episode hypomanic, Bipolar I Disorder single manic episode in full remission, Bipolar I Disorder single manic episode in partial remission, Bipolar I Disorder single manic episode (mild), Bipolar I Disorder single manic episode (moderate), Bipolar I Disorder single manic episode (severe with psychotic features), Bipolar I Disorder single manic episode severe without psychotic features, Bipolar I Disorder single manic episode (unspecified), Bipolar II Disorder, Body Dysmorphic Disorder, Borderline Personality Disorder, Breathing- Related Sleep Disorder, Brief Psychotic Disorder, Bulimia Nervosa, Circadian Rhythm Sleep Disorder, Conversion Disorder, Cyclothymic Disorder, Delusional Disorder, Dependent personality Disorder, Depersonalization disorder, Depressive Disorder NOS, Dissociative Amnesia, Dissociative Disorder NOS, Dissociative fugue, Dissociative Identity Disorder, Dissociative Disorders, Dyspareunia, Dyssomnia NOS, Dyssomnia Related to (Another Disorder), Dysthymic Disorder, Eating disorder NOS, Exhibitionism, Female Dyspareunia Due to Medical Condition, Female Hypoactive Sexual Desire Disorder Due to Medical Condition, Female Orgasmic Disorder, Female Sexual Arousal Disorder, Fetishism Sexual Disorders, Frotteurism Sexual Disorders, Gender Identity Disorder in Adolescents or Adults, Gender Identity Disorder in Children, Gender Identity Disorder NOS, Generalized Anxiety Disorder, Histrionic Personality Disorder, Hypoactive Sexual Desire Disorder, Hypochondriasis, Impulse - Control Disorder NOS, Insomnia Related to Another Disorder, Intermittent Explosive Disorder, Kleptomania, Major Depressive Disorder Recurrent (in full remission), Major Depressive Disorder Recurrent (in partial remission), Major Depressive Disorder Recurrent (Mild), Major Depressive Disorder recurrent (moderate), Major Depressive Disorder recurrent (severe with psychotic features), Major Depressive Disorder, Recurrent (severe without psychotic features), Major Depressive Disorder Recurrent (unspecified), Major Depressive Disorder Single Episode (in full remission), Major Depressive Disorder single episode (in partial remission), Major Depressive Disorder single episode (Mild), Major Depressive Disorder single episode, Major Depressive Disorder single episode (severe with psychotic features), Major Depressive Disorder single episode (severe without psychotic features), Major Depressive Disorder single episode (unspecified), Male Dyspareunia Due to Medical Condition, Male Erectile Disorder, Male Erectile Disorder Due to Medical Condition, Male Hypoactive Sexual Desire Disorder Due to Medical Condition, Male Orgasmic Disorder, Mood Disorder Due to Medical Condition, Narcissistic Personality Disorder, Narcolepsy Sleep Disorders, Nightmare Disorder Sleep Disorders, Obsessive Compulsive Disorder, Obsessive-Compulsive Personality Disorder, Other Female Sexual Dysfunction Due to Medical Condition, Other Male Sexual Dysfunction Due to Medical Condition, Pain Disorder Associated with both Psychological Factors and Medical Conditions, Pain Disorder Associated with Psychological Features, Panic Disorder with Agoraphobia, Panic Disorder without Agoraphobia, Paranoid Personality Disorder, Paraphilia NOS, Parasomnia NOS, Pathological Gambling, Pedophilia Sexual Disorders, Personality Disorder NOS, Posttraumatic Stress Disorder, Premature Ejaculation, Primary Hypersomnia, Primary Insomnia, Psychotic Disorder Due to Medical Condition with Delusions, Psychotic Disorder Due to Medical Condition with Hallucinations, Psychotic Disorder NOS, Pyromania, Schizoaffective Disorder, Schizoid Personality Disorder, Schizophrenia Catatonic Type, Schizophrenia Disorganized Type, Schizophrenia Paranoid Type, Schizophrenia Residual Type, Schizophrenia Undifferentiated Type, Schizophreniform Disorder, Schizotypal Personality Disorder, Sexual Aversion Disorder, Sexual Disorder NOS, Sexual Dysfunction NOS, Sexual Masochism, Sexual Sadism, Shared Psychotic Disorder, Sleep Disorder Due to A Medical Condition Hypersomnia Type, Sleep Disorder Due to A Medical Condition Insomnia Type, Sleep Disorder Due to A Medical Condition Mixed Type, Sleep Disorder Due to A Medical Condition Parasomnia Type, Sleep Terror Disorder, Sleepwalking Disorder, Social Phobia, Somatization Disorder, Somatoform Disorder NOS, Specific Phobia, Trichotillomania, Undifferentiated Somatoform Disorder, Vaginismus Sexual Disorders, and Voyeurism.

It will also be appreciated that the detection method of the invention may be used to detect the presence of an inflammatory disorder, i.e. diagnosis of an inflammatory disorder. Thus, according to a further aspect of the invention, there is provided a method of diagnosing an inflammatory disorder, which comprises the method as hereinbefore defined. In one embodiment, the inflammatory disorder is selected from: allergic reaction, ankylosing spondylitis, appendicitis, asthma (such as chronic asthma and allergic asthma), Crohn's disease, dermatomyositis, diabetes mellitus type 1, diarrhoea (such as diarrhoea caused by *E. coli*), haemorrhagic shock, inflammations of the airways, skin and gastrointestinal tract, inflammatory bowel diseases, inflammatory lesions, lupus erythematosus, lupus nephritis, metastases, osteomyelitis, paroxysmal nocturnal hemoglobinuria, polymyositis, Pseudomonas aeruginosa infection, psoriasis, psoriatic arthritis, rheumatic diseases (such as rheumatoid arthritis), sepsis (such as sepsis caused by Gram-negative bacteria), severe allergic disorders, SLE, stroke, systemic lupus erythematosus, systemic scleroderma, thromboembolism and ulcerative colitis.

When the method of the invention is used in the diagnosis of an inflammatory disorder the first and/or second binding agents may comprise any of the monoclonal antibodies listed below in Table 5:

**Table 5: Examples of Monoclonal Antibodies as First and/or Second Binding Agents for use in the diagnosis of an inflammatory disorder**

| ***Name*** | ***Trade Name*** | ***Type*** | ***Source*** | ***Target*** | ***Use*** |
|---|---|---|---|---|---|
| Adalimumab | Humira | mab | human | TNF-α | rheumatoid arthritis etc. |
| Afelimomab | | F(ab') 2 | mouse | TNF-α | sepsis |
| ALD518 | | | humanized | IL-6 | rheumatoid arthritis |
| Atlizumab | (Ro)Acte mra | mab | humanized | IL-6 receptor | rheumatoid arthritis |
| Benralizumab | | mab | humanized | CD125 | asthma |
| Bertilimumab | | mab | human | CCL11 | severe allergic disorders |
| Besilesomab | Scintimun | mab | mouse | CEA-related antigen | inflammatory lesions and metastases-detection |
| Biciromab | FibriScint | Fab' | mouse | fibrin II-beta | thromboembolism -diagnosis |
| Briakinumab | | mab | human | IL-12, IL-23 | psoriasis, rheumatoid arthritis, inflammatory bowel diseases |
| Canakinumab | Ilaris | mab | human | IL-1 | rheumatoid arthritis |
| Cedelizumab | | mab | humanized | CD4 | treatment of autoimmune diseases |
| Certolizumab pegol | Cimzia | Fab' | humanized | TNF-α | Crohn's disease |
| Clenoliximab | | mab | chimeric | CD4 | rheumatoid arthritis |
| Eculizumab | Soliris | mab | humanized | C5 | paroxysmal nocturnal hemoglobinuria |
| Edobacomab | | mab | mouse | endotoxin | sepsis caused by Gram-negative bacteria |
| Efalizumab | Raptiva | mab | humanized | LFA-1 (CD11a) | psoriasis-blocks T-cell migration |
| Efungumab | Mycograb | scFv | human | Hsp90 | invasive Candida infection |
| Epratuzumab | | mab | humanized | CD22 | SLE |
| Erlizumab | | F(ab') 2 | humanized | ITGB2-CD18 | stroke |
| Fanolesomab | Neutro Spec | mab | mouse | CD15 | appendicitisdiagnosis |
| Fezakinumab | | mab | human | IL-22 | rheumatoid arthritis, psoriasis |
| Fontolizumab | HuZAF | mab | humanized | IFN-γ | Crohn's disease etc. |
| Golimumab | Simponi | mab | human | TNF-α | rheumatoid arthritis, psoriatic arthritis, ankylosing spondylitis |
| Gomiliximab | | mab | chimeric | CD23-IgE receptor | allergic asthma |
| Infliximab | Remicade | mab | chimeric | TNF-α | rheumatoid arthritis, ankylosing spondylitis, psoriatic arthritis, psoriasis, Crohn's disease, ulcerative colitis |
| Keliximab | | mab | chimeric | CD4 | chronic asthma |
| Lebrikizumab | | mab | humanized | IL-13 | asthma |
| Mepolizumab | Bosatria | mab | humanized | IL-5 | asthma and white blood cell diseases |
| Metelimumab | | mab | human | TGF beta 1 | systemic scleroderma |
| Natalizumab | Tysabri | mab | humanized | integrin α4 | Crohn's disease |
| Nebacumab | | mab | human | endotoxin | sepsis |
| Ocrelizumab | | mab | humanized | CD20 | rheumatoid arthritis, lupus erythematosus etc. |
| Odulimomab | | mab | mouse | LFA-1-CD11a | immunological diseases |
| Omalizumab | Xolair | mab | humanized | IgE Fc region | allergic asthma |
| Otelixizumab | | mab | chimeric/hu manized | CD3 | diabetes mellitus type 1 |
| Pagibaximab | | mab | chimeric | lipoteichoic acid | sepsis (Staphylococcus) |
| Panobacumab | | mab | human | Pseudomo nas aeruginos a | Pseudomonas aeruginosa infection |
| Pascolizumab | | mab | humanized | IL-4 | asthma |
| Priliximab | | mab | chimeric | CD4 | Crohn's disease |
| Reslizumab | | mab | humanized | IL-5 | inflammations of the airways, skin and gastrointestinal tract, some autoimmune disorders |
| Rontalizumab | | mab | humanized | IFN-α | systemic lupus erythematosus |
| Rovelizumab | Leuk Arrest | mab | humanized | CD11, CD18 | haemorrhagic shock etc. |
| Ruplizumab | Antova | mab | humanized | CD154-CD40L | rheumatic diseases |
| Sifalimumab | | mab | humanized | IFN-α | SLE, dermatomyositis, polymyositis |
| Siplizumab | | mab | humanized | CD2 | psoriasis |
| Sulesomab | Leuko Scan | Fab' | mouse | NCA-9(granulocyte antigen) | osteomyelitis (imaging) |
| Talizumab | | mab | humanized | IgE | allergic reaction |
| Teplizumab | | mab | humanized | CD3 | diabetes mellitus type 1 |
| TGN1412 | | | humanized | CD28 | rheumatoid arthritis |
| Tocilizumab | (Ro)Acte mra | mab | humanized | IL-6 receptor | rheumatoid arthritis |
| Toralizumab | | mab | humanized | CD154-CD40L | rheumatoid arthritis, lupus nephritis etc. |
| Urtoxazumab | | mab | humanized | E coli | diarrhoea caused by E. coli |
| Ustekinumab | Stelara | mab | human | IL-12, IL-23 | psoriasis, psoriatic arthritis |
| Vedolizumab | | mab | humanized | integrin α4β7 | Crohn's disease, ulcerative colitis |
| Vepalimomab | | mab | mouse | AOC3 (VAP-1) | inflammation |
| Visilizumab | Nuvion | mab | humanized | CD3 | Crohn's disease, ulcerative colitis |
| Zanolimumab | HuMax-CD4 | mab | human | CD4 | rheumatoid arthritis,psoriasis |
| Zolimomab aritox | | mab | mouse | CD5 | systemic lupus erythematosus |

It will also be appreciated that the detection method of the invention may be used to detect the presence of macular degeneration, i.e. diagnosis of macular degeneration. Thus, according to a further aspect of the invention, there is provided a method of diagnosing macular degeneration, which comprises the method as hereinbefore defined.

When the method of the invention is used in the diagnosis of macular degeneration the first and/or second binding agents may comprise any of the monoclonal antibodies listed below in Table 6:

**Table 6: Examples of Monoclonal Antibodies as First and/or Second Binding Agents for use in the diagnosis of macular degeneration**

| ***Name*** | ***Trade Name*** | ***Type*** | ***Source*** | ***Target*** | ***Use*** |
|---|---|---|---|---|---|
| Lerdelimumab | | mab | human | TGF beta 2 | reduction of scarring after glaucoma surgery |
| Ranibizumab | Lucentis | Fab | humanized | VEGF-A | macular degeneration (wet form) |
| Sonepcizumab | | | humanized | sphingosine -1-phosphate | choroidal and retinal neo-vascularization |

It will also be appreciated that the detection method of the invention may be used to detect the presence of transplant rejection, i.e. diagnosis of transplant rejection. Thus, according to a further aspect of the invention, there is provided a method of diagnosing transplant rejection, which comprises the method as hereinbefore defined.

When the method of the invention is used in the diagnosis of transplant rejection the first and/or second binding agents may comprise any of the monoclonal antibodies listed below in Table 7:

**Table 7: Examples of Monoclonal Antibodies as First and/or Second Binding Agents for use in the diagnosis of transplant rejection**

| ***Name*** | ***Trade Name*** | ***Type*** | ***Source*** | ***Target*** | ***Use*** |
|---|---|---|---|---|---|
| Basiliximab | Simulect | mab | chimeric | CD25-α chain of IL-2 receptor | prevention of organ transplant rejections |
| Cedelizumab | | mab | humanize d | CD4 | prevention of organ transplant rejections |
| Daclizumab | Zenapax | mab | humanize d | CD25-α chain of IL-2 receptor | prevention of organ transplant rejections |
| Gavilimomab | | mab | mouse | CD147-basiqin | graft versus host disease |
| Inolimomab | | mab | mouse | CD25-α chain of IL-2 receptor | graft versus host disease |
| Muromonab-CD3 | Orthoclone OKT3 | mab | mouse | CD3 | prevention of organ transplant rejections |
| Odulimomab | | mab | mouse | LFA-1 (CD11a) | prevention of organ transplant rejections |
| Siplizumab | | mab | humanize d | CD2 | graft-versus-host disease (prevention) |
| Zolimomab aritox | | mab | mouse | CD5 | graft-versus-host disease |

Example of other monoclonal antibodies which may be used as first and/or second binding agents include those listed below in Table 8:

**Table 8: Examples of Monoclonal Antibodies as First and/or Second Binding Agents**

| ***Name*** | ***Trade Name*** | ***Type*** | ***Source*** | ***Target*** | ***Use*** |
|---|---|---|---|---|---|
| Atorolimumab | | mab | human | Rhesus factor | hemolytic disease of the newborn |
| Bapineuzumab | | mab | humanized | beta amyloid | Alzheimer's disease |
| Catumaxomab | Removab | 3funct | rat/mouse | EpCAM, CD3 | malignant ascites |
| Denosumab | Prolia | mab | human | RANKL | osteoporosis |
| Fresolimumab | | mab | human | TGF-β | idiopathic pulmonary fibrosis, focal segmental glomerulosclero sis |
| Gantenerumab | | mab | human | beta amyloid | Alzheimer's disease |
| Raxibacumab | | mab | human | anthrax toxin protective antigen | anthrax-prophylaxis and treatment |
| Stamulumab | | mab | human | myostatin | muscular dystrophy |
| Tanezumab | | mab | humanized | NGF | pain |
| Tefibazumab | Aurexis | mab | humanized | clumping factor A | Staphylococcus aureus infection |

It will also be appreciated that the detection method of the invention may be used as a 'companion diagnostic' to a therapeutic method, such as a drug or device, in order to justify the use of the therapeutic. Companion diagnostics are increasingly finding utility in the justification of expensive or high risk treatments which only confer benefit to a subset of the population. The detection method of the invention is especially well-suited to such purposes owing to its specificity, and the automated nature of the detection and consequent rapidity of results.

It will also be appreciated that the detection method of the invention may be used to screen a small molecule library for one or more small molecules of interest. Small molecule libraries of the type useful in methods of the invention may be formed by methods well known in the art or may be obtained commercially (e.g., ChemBridge). Small molecule libraries include combinatorial libraries. A combinatorial library can be a library of molecules containing a large number, typically between 10³ and 10⁶ different-sequence oligomers, typically characterized by different sequences of subunits, or a combination of different sequences of side chains and linkages, or different- substituent compounds. Various solid-phase or solution-phase synthetic methods for preparing small molecule libraries are known.

It will also be appreciated that the detection method of the invention may be used to detect the presence of small molecules such as antibiotics (i.e. tetracycline or gentamycin), contaminants (i.e. dioxin), pollutants, steroids (i.e. corticosteroids), oncopharm (MTX), paracetamol or NABQI present within given samples. In one embodiment, the sample may comprise a food stuff such as milk. In one embodiment, the analyte may comprise a small molecule such as a steroid (i.e. a corticosteroid) and the sample may comprise a blood or urine sample extracted from an athlete.

It will also be appreciated that the detection method of the invention may be used to screen for fetal abnormalities or pregnancy related illnesses, such as pre-eclampsia. Thus, according to a further aspect of the invention, there is provided a method of diagnosing fetal abnormalities or pregnancy related illnesses, such as pre-eclampsia, which comprises the method as hereinbefore defined.

Conventional tests for chromosome abnormalities of foetuses are invasive because they require a medical professional to obtain cell material directly, from the foetus or amniotic fluid surrounding the foetus. Such invasive procedures can lead to complications or even termination of the pregnancy.

It is desirable to provide a test for fetal numerical chromosome abnormality and/or gene dosage differences which is non-invasive, for example, by testing a sample of maternal blood, which will have fetal nucleic acid present in it. Fetal DNA can be found in maternal blood plasma early in pregnancy and its transplacental transition appears to be increased in cases of fetal chromosome abnormalities. However, it can be difficult to detect an imbalance in chromosome and/or gene copy numbers using maternal blood because the increased or decreased copy number of the target gene or chromosome observed in abnormal fetal DNA is likely to be masked by the presence of normal maternal DNA in excess. Conventional quantitative real time PCR is not sensitive enough to detect the small difference in gene or chromosome copy number, because only a small fraction of the template DNA is from the fetus.

The detection method of the invention provides an effective method for detecting surface analytes present on fetal blood cells, such as trophoblasts. In one embodiment, the surface antigen is HLA-G. HLA-G has previously been shown to be a surface antigen which is expressed in trophoblasts isolated from the maternal circulation (Guetta et al (2005) Journal of Histochemistry & Cytochemistry 53(3), 337-339).

According to a further aspect of the invention there is provided an analyte detection kit which comprises one or more magnetic complexes comprising a magnetic particle coated with a DNA or RNA oligonucleotide capable of specific binding to the analyte. In one embodiment, the kit additionally comprises instructions to use the kit in accordance with the methods defined hereinbefore.

The invention will now be illustrated with reference to the following non-limiting examples:

### Example 1: Detection of Thrombin in a ViBE Bioanalyzer

The aim of this study was to demonstrate detection of thrombin utilizing an aptamer sandwich assay in a ViBE Bioanalyzer (obtained from Bioscale; hereinafter referred to as Apta-ViBE). Increasing concentrations of thrombin will translate as amplified acoustic membrane microparticle detection (AMMP) response units (RU).

The sandwich assay consists of streptavidin beads which will be coupled to a biotinylated thrombin 15mer aptamer (B15aptamer) This aptamer binds to exosite I (HD1, Fibrinogen recognition site) of thrombin. There is another thrombin 29mer aptamer which is fluoresceinated (F29aptamer) and this binds to exosite II (HD22, Heparin binding site) of thrombin. When thrombin is detected, it will bind to both aptamers at their separate sites so completing the sandwich link. The fluorescein on the F29aptamer will bind to the anti-fluorescein antibody located on the BioScale chip when the sample mixture is run through the Apta-ViBE.

Absence of thrombin (protein delete) and substitution of thrombin with dilutions of Factor IXa should demonstrate no signal increase from baseline in AMMP RU and will be negative control samples.

Interleukin-6 (IL-6) protein detection has been characterised on the ViBE bioanalyzer and is used as a positive control to check each cartridge and each channel/sensor is functioning properly. The IL-6 kit contains a set of standard protein dilutions which can be incubated with beads and fluorescein-tagged IL-6 antibody in order to be detected when run through the ViBE bioanalyzer.

### Materials and Methods

### 1. Resuspend Oligonucleotides

### Aim

Oligonucleotides are shipped lyophilized to retain stability. Each time a new oligo arrives resuspend in TE Buffer, aliquot into separate tubes to prevent freeze-thaw cycles and store at -20°C. Oligos are stable at -20°C for at least 6 months and in solution at 4°C for at least 2 weeks (although they are usually fully viable for much longer than these times).

### Ma terials

- Lyophilised oligonucleotide
- Tris-EDTA Buffer (TE Buffer)

### Method

- Centrifuge the vial to get all oligo at bottom of tube
- Dilute to 100µM with TE Buffer. Take the number of nmols provided on data sheet, move the decimal point one place to the right to get the number of µl of TE Buffer to add
- Vortex for 1 min to mix thoroughly
- Pipette 5µl aliquots into DNAse-free microcentrifuge tubes, label and store at -20°C
-

### 2. Bind Biotinylated Aptamer to Streptavidin Beads

### Aim

The magnetic beads are coated with streptavidin which binds with very high affinity to biotin. Incubate the streptavidin beads with the biotinylated 15mer aptamer in order to conjugate the aptamer to the bead. The optimum binding capacity of aptamer is used in order to saturate as many of the available streptavidin binding sites as possible. Then incubate the bead complex with a bead blocker such as biotin-BSA in order to prevent non-specific binding to the bead of other proteins. Wash the beads between each step in order to remove any unbound particles from the bead complex. This binding step is best done 2-3 days before assay day to enable full blockage of non-specific binding sites on streptavidin beads with biotin-BSA.

### Ma terials

- Streptavidin Beads (BioScale-10mg/ml)
- Magnet - (DynaMag^{™}-2)
- Biotinylated 15mer (B15aptamer)
- Bead Blockers - (Biotin-BSA or free biotin)
- Disposable Plastic Pipettes (narrow tip)
- Washing Buffer (THST)
- Binding Buffer (THS)
- BioScale Bead Storage Buffer

### Method

- Ensure Washing Buffer (THST) and Binding Buffer (THS) are made up (see step 3 for details).
- Vortex streptavidin beads in the original vial for 2 mins to resuspend them. Intermittently flick bottom of tube to release all beads. This is critical so the beads are dispersed before coupling
- Calculate volume of beads to be used in the assay. BioScale beads are suspended as 10mg (6-7 x 10⁸) beads/ml. Using 1mg (0.1ml) first will couple enough beads to be used for 5 x 1:100 dilutions, each of which can do 1 bead count and 4 cartridge runs. 1mg of beads binds with up to 200 picomoles of single-stranded nucleotide (binding capacity inversely related to molecule size). This is for DynaBeads from Invitrogen data sheet. BioScale have confirmed the beads they source have the same specification. 10mg (1ml) beads can be used when a formulation is reached and ~50 cartridges will be run.
- Add 0.1 ml beads to a sterile microcentrifuge tube and place tube on magnet for 2 min using a timer. It is important to allow the beads to sit against the magnet for at least 2 min to ensure they are all collected on the side of the tube
- Remove the supernatant with a sterile plastic pipette whilst tube is still on magnet. Use a narrow pipette rather than a wide one so as to not disrupt the beads. Avoid bubbles throughout when pipetting
- Take the rack with the beads off the magnet. Then add 1ml Washing Buffer along the inside of the tube where the beads are collected. Vortex for 30 sec, centrifuge for 10 sec to get all the beads off the cap, place on magnet for 2 min and repeat wash for a total of 3 times
- After the third wash place tube on the magnet for 2 min and remove all Washing Buffer
- Add 200 picomoles (2µl of 0.1nmol/µl or 100µM solution) of B15aptamer to 300µl of Binding Buffer in a clean, empty eppendorf tube. Mix and then add solution to the eppendorf containing the beads
- Incubate B15aptamer and beads on vortex shaker for 1 hour at room temperature (RT)
- Place tube on magnet for 2 min and wash beads with 1ml Washing Buffer 3 times as before
- Resuspend in 1ml Binding Buffer
- Add 10ug Bt-BSA per mg of beads (5µl of 1985µg/ml stock solution) (or instead add 35µg free biotin per mg of beads) and incubate for 30 min, while vortexing at the same time at a constant speed (RT)
- Place tube on magnet for 2 min and wash beads with 1ml Washing Buffer 3 times as before
- Resuspend the beads in ½ volume, i.e. 50µl of Bead Storage Buffer (or PBS 0.1% BSA)

### 3. Prepare Buffers

### Aim

To have all necessary buffers ready for assay. Typically they should be prepared at least one day before in order that there is time to autoclave, cool and degas overnight. This step will need to be done before step 2 if there is no stock buffer already made up.

### Materials

- Trizma ® Base (Sigma) 121.14 g/mol
- Concentrated HCl (10M)
- NaCl 58.44 g/mol
- KCl 74.55 g/mol
- MgCl₂ 203.30 g/mol (note: this is the hydrated NOT anhydrous form, either can be used but the differing molar masses must be taken into account)
- CaCl₂ 110.99 g/mol
- Tween®20
- Glass Bottles (1L, 500ml and 100ml)
- Tin Foil
- Autoclave Tape
- Autoclave
- Pump with Tubing for Degassing
- Magnetic Followers
- Stir Plate
- Nalgene 500ml Bottle Top 0.2µm Filters

### Make Up 10X Tris-HCl + Salts (THS)

- Stir in 60.55g Trizma ® Base to about 900ml Ultrapure H₂O in a glass bottle with a magnetic stirrer
- Using a pH meter, add concentrated HCl dropwise, to titrate solution to pH 7.4
- Add whilst stirring: 58.44g NaCl; 3.73g KCl; 2.03g MgCl₂ and 5.55g CaCl₂
- Make up to 1 litre with Ultrapure H₂O
- Apply lid loosely, cover with foil, secure with autoclave tape and autoclave
- When cooled vacuum filter through 0.2µm filter into a sterile bottle. This is the stock 10 X THS

### Autoclave Bottles

- Rinse 4 x 1L bottles and 4 x 500ml bottles in distilled water, cover lids with foil and autoclave
- Rinse and autoclave magnetic followers

### Make up Tris-HCl + Salts + 0.05% Tween (THST) (Washing Buffer)

- Add 10ml Tween®20 to 90ml Ultrapure water in an autoclaved bottle. This is the stock 10% Tween®20
- Add 100ml of the autoclaved 10 X THS, 795ml Ultrapure water and 5ml 10% Tween®20 to a 1 litre bottle
- Stir for 10 mins with magnetic follower and stirring plate
- Vacuum filter through 0.2µm filter into a sterile bottle and degas overnight. (This is the Washing Buffer. 10% BioScale Diluent to be added on day of assay to make up Running Buffer)

### 4. Count The Beads

### Aim

The optimum concentration of beads is the one which forms a full monolayer over the BioScale chip without bead stacking. This optimum concentration has been determined by BioScale to be 1.5 x 10⁵ beads/ml, which is the final concentration in each well of the Corning assay plate. Typically, as in the IL-6 assay, 40µl of bead solution is added to 80µl of sample analyte which results in a final volume of 120µl per well. Therefore, the bead lots which are pipetted into the well plates must be at 3 x concentration, i.e. at 4.5 x 10⁵ beads/ml, so when added to the samples they are at the final concentration of 1.5 x 10⁵ beads/ml.

Determine the starting concentration of each lot of aptamer-bead conjugate made in step 2 in order to calculate the volume of beads to add to diluent to make the 3 x concentration solution. A coulter counter can be used but BioScale recommend using the ViBE bioanalyzer to determine bead concentration. The more beads that are run over the chip, the higher the accumulation value is. Run beads of known concentration (control beads) through the ViBE first at several dilutions to plot accumulation value against bead concentration. Then run each aptamer-bead lot through at several dilutions and, based on the accumulation values, determine the concentration of each aptamer-bead lot.

The optimum dilution of beads will give an optimum accumulation value of between 1200 and 1800 parts per million (ppm). Once the bead concentration and optimum dilution is determined it is used for the entire aptamer-bead lot. The bead count can be performed on the day of the assay or beforehand if there is a very long experiment. Allow at least 4 hours, although if done on the day of assay it does includes the initial IL-6 control which is required for the assay.

### Materials

- Running Buffer (degassed for at least 24 hours)
- Washing Buffer (degassed for at least 24 hours)
- Regeneration Buffer
- BioScale Diluent (PBS and Fetal Bovine Serum, pH 7.0)
- 10 X THS
- Magnetic Beads (bound to B15aptamer)
- Control Beads (must have a known concentration)
- IL-6 Kit
- Corning 96 Well Assay Plate with Non-Binding Surface (cat. 3605)
- Plate Sealing Tape
- Plate Tape Roller
- Solution Basins 55ml
- BioScale Cartridge
- BioScale Bead Counting Excel Template

### Method

### Prepare IL-6 controls

- Prepare IL-6 beads by vortexing the tube of IL-6 bead stock for at least 1 min, inverting and flicking the tube every 20 sec
- To a 15ml tube containing 3ml of BioScale Diluent, add 4.5 x 10⁵ IL-6 beads/ml (102.27µl of batch TK001: IL-6 reagent concentrations will vary between batches of IL-6 kits so check the correct volumes to add with each kit). For each 1ml of BioScale Diluent the bead solution is required to have a final concentration of 4.5 x 10⁵ beads/ml, which will be at the optimum concentration of 1.5 x 10⁵ beads/ml when diluted 3 fold after adding to the 80µl of standard in each well

- Vortex for 10 sec then add 300ng/ml IL-6 fluorescein-antibody (2.86µl of batch TK001: add correct volume accordingly for different batches). For each 1ml of BioScale Diluent the fluorescein-antibody is required to have a final concentration of 300ng/ml, which will be at 100ng/ml after adding to the standards in each well
- Vortex for 10 sec then rotate end-over-end for at least 5 min

### Prepare Bead Solutions

- Vortex the aptamer-bead lot/s being tested and the control beads for at least 1 min to ensure that proper mixing and re-suspension of beads is achieved. While vortexing, invert and flick side of tube every 20 sec
- Make up 50ml of THS by adding 5ml of 10X THS to 45ml of Ultrapure water
- Prepare 1:100 dilutions of the bead lot/s being tested and control beads by adding 10µl of bead stock to 990µl of THS. Vortex well to ensure proper mixing and resuspension
- To a 15ml tube containing 5ml of THS, add 200µl of the 1:100 control bead stock. Repeat for each aptamer-bead lot to be counted
- Vortex for 10 sec then rotate end-over-end for at least 5 min
- Note: F29aptamer/FI-Ab does not need to be added since the bead counting protocol does not require detecting any positive controls

### Prepare 96-Well Plates

• In order to accurately measure the concentration of a bead stock, several dilutions of the 5ml bead solutions must be made. Start with the control beads which is a bead stock of known concentration and which the bead count can be based on. The IL-6 beads can be used as control beads
• As shown in Table 9 below, for every bead lot, including the control, prepare 4 dilutions in duplicate from column 4 of a 96-well plate. Columns 1-3 (and 6-8) are IL-6 controls to demonstrate the chip is functioning correctly

**Table 9**

| Sensor # | Volume of Aptamer Diluent (µl) | Volume of 5ml Bead Solution (µl) |
|---|---|---|
| 1 | 100 | 20 |
| 2 | 100 | 20 |
| 3 | 90 | 30 |
| 4 | 90 | 30 |
| 5 | 80 | 40 |
| 6 | 80 | 40 |
| 7 | 70 | 50 |
| 8 | 70 | 50 |

• Add THS (Aptamer Diluent) to the 96-well plate first and then add the bead solution (i.e. 20µl, 30µl, etc.) Pipette concentrations from low to high so that the same pipette can be used for each solution. Use column 4 for the control beads and column 5 for the aptamer beads (see plate layout in Table 10 below)
• Add 80µl BioScale Diluent to each well of the IL-6 negative column and 80µl of each IL-6 calibrant to the appropriate wells in the IL-6 Curve and Inverse Curve columns(see plate layout in Table 10 below)
• Pour out the IL-6 bead/fluorescein-antibody solution into a solution basin
• Dispense 40 µl of IL-6 bead/fluorescein-antibody solution into each IL-6 column
• The remaining columns on the plate can be used to start the aptamer assay once the bead count is complete and it is known what volume of beads added gives the optimum signal 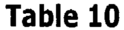 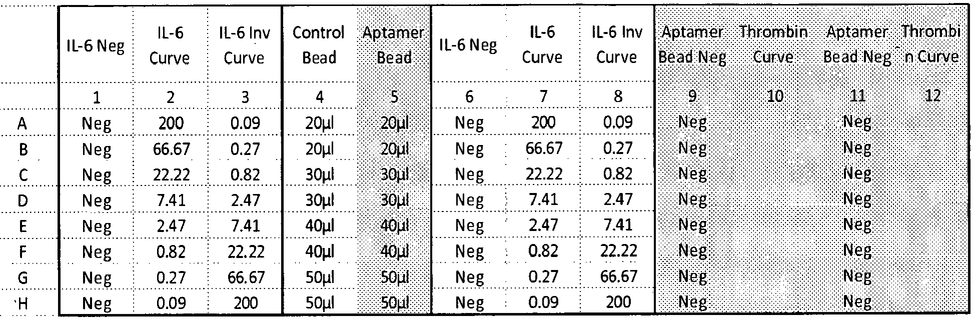
• Place a plate sealing tape over the plate and secure the tape using a roller
• Place the plate on the Big Bear shaker, turn on and set at a speed of 1200 rpm. It is important to keep the beads shaking at all times during incubation so they do not settle. Avoid stacking plates on the orbital shaker wherever possible and ideally use 1 shaker per well plate used
• Incubate for 3 hours for the IL-6 controls in colums 1-3
• Start up the Apta-ViBE around 2 hours before the first column of samples is ready to be run

### Determine the Bead Count

- Run the IL-6 controls followed by the control beads and aptamer bead lots to be counted through the Apta-ViBE - see section 6 for full details
- Once the aptamer bound beads have run through, look at the bead accumulation values for the increasing bead concentrations. The optimum concentration of beads to be used is the one which gives an accumulation value of between 1200 and 1800 ppm. For example, using the results in Table 11 below the 40µl bead (+80µl Aptamer Diluent) gives a bead accumulation of around 1500 ppm so is therefore the optimum dilution for this lot of B15 aptamer-bead complex 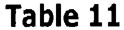 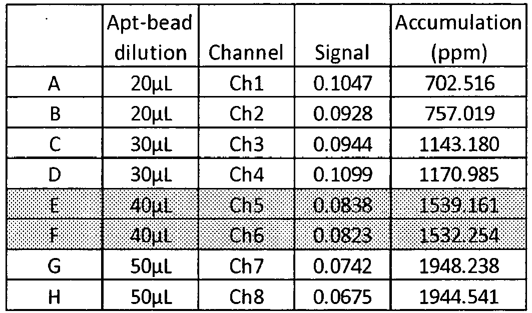
- To more accurately count the beads, the exact concentration of aptamer-bead lots can be found using the BioScale Bead Counting Excel Template. Once all the bead lots have been run copy the raw data from the C:BioScale/Data folder and paste it into the results tab of the Excel sheet
- Input the exact concentration of the control beads and the plate layout used for the bead count
- Click on the "analyze" button
- The template will plot a straight line graph of accumulation value against control bead concentration. The concentration of the aptamer-bead lots can then be calculated from the equation of this graph using the accumulation values generated in the bead count

### 5. Incubate Thrombin with Aptamers

### Aim

Once the optimum concentration of B15 aptamer-bead complex has been determined, mix together with the F29 aptamer in a tube and then incubate with the thrombin standard dilutions in 96 well plates placed on the orbital shaker for the desired amount of time. The Corning 96 well plates have a nonionic hydrophilic surface which minimises molecular interactions, i.e. adsorption, of the bead-aptamer-thrombin complex to the plate surface. During incubation the aptamers will bind the thrombin creating a sandwich of magnetic bead-streptavidin-biotin-15mer aptamer-thrombin-29mer aptamer-fluorescein.

### Materials

- THS
- B15 aptamer-bead complex
- Aptamer (oligonucleotide) - Fluoresceinated 29mer (F29-aptamer)
- Thrombin (store at -20°C, place on ice 5-10 mins to reduce viscosity before diluting)
- Factor IXa (negative control) (store at -20°C, place on ice 5-10 mins to reduce viscosity before diluting)
- IL-6 Kit
- Corning 96 Well Assay Plate with Non-Binding Surface (cat. 3605)
- Plate Sealing Tape
- Plate Tape Roller
- Solution Basins 55ml

### Method

### Prepare B15Aptamer-bead Complex/F29Aptamer Solution

- Vortex the tube of the 1:100 dilution of B15 aptamer-bead complex for 1 min, flicking the tube to ensure all beads are resuspended
- To a 15 ml tube containing 5ml THS add 200µl (or modify if an exact bead count) of the 1:100 dilution of bead stock and vortex for 10 sec
- Add 2 µl of 0.1nmol/µl or 100µM solution of F29 aptamer to tube, vortex for 30 sec and rotate end-over-end for at least 5 min

### Prepare IL-6 Bead/Antibody Solution

- Prepare IL-6 beads by vortexing the tube of IL-6 bead stock for at least 1 min, inverting and flicking the tube every 20 sec
- To a 15ml tube containing 3ml of BioScale Diluent, add 4.5 x 10⁵ IL-6 beads/ml (102.27µl of batch TK001: IL-6 reagent concentrations will vary between batches of IL-6 kits so check the correct volumes to add with each kit)
- Vortex for 10 sec then add 300ng/ml IL-6 fluorescein-antibody (2.86µl of batch TK001: add correct volume accordingly for different batches
- Vortex for 10 sec then rotate end-over-end for at least 5 min

### Prepare Thrombin/FactorIXa Standard Dilutions

The desired detection range of thrombin is from 2000ng/ml down to less than 10pg/ml.
• Thrombin initial stock is 8.9mg/ml; current stock is 8.1mg/ml (lot no. Z0602-0.1MG)
• Factor IXa current stock is 4.7mg/ml
• Each assay plate well will use 80µl of each thrombin standards which will be added to the 40µl of SA bead-B15aptamer/F29aptamer mixture
• Initial set of dilutions for the thrombin assay standards are 2 high concentrations and a range of 6 lower concentrations. Each set of standards should be inverted to check equal functionality of sensors, e.g. plate layout in Table 12 below:

**Table 12**

| | ThrNeg | | ThrCurve | | ThrInvCurv |
|---|---|---|---|---|---|
| A | Thr neg | S3 | 9.259 | S8 | 0.009 |
| B | Thr neg | S4 | 2.315 | S7 | 0.036 |
| C | Thr neg | S5 | 0.579 | S6 | 0.145 |
| D | Thr neg | S6 | 0.145 | S5 | 0.579 |
| E | Thr neg | S7 | 0.036 | S4 | 2.315 |
| F | Thr neg | S8 | 0.009 | S3 | 9.259 |
| G | Thr neg | S2 | 333.333 | S1 | 2000.000 |
| H | Thr neg | S1 | 2000.000 | S2 | 333.333 |

• To make a 2000ng/ml thrombin dilution add 0.677µl thrombin to 3ml diluent or buffer in a DNAse free tube and vortex for 30 sec. This is Standard 1 (S1)
• To make 333.333ng/ml (6x dilution) add 0.5ml of Standard 1 to 2.5ml diluent or buffer and vortex for 30 sec. This is Standard 2
• To make 9.259ng/ml (36x dilution) add 0.5ml of Standard 2 to 2.5ml diluent or buffer and vortex for 30 sec. Add 0.5ml of this dilution to another 2.5ml diluent or buffer and vortex for 30 sec. This is Standard 3
• To make 2.315ng/ml (4x dilution) add 0.25ml of Standard 3 to 0.75ml diluent or buffer and vortex for 30 sec. This is Standard 4
• Continue with 4x dilutions to create Standards 5-8
To investigate intermediate concentrations make up alternative dilutions as required, e.g. Standard 1 at 1000ng/ml thrombin (add 0.339µl to 3ml diluent or buffer or 0.370µl of current stock to 3ml) with serial 3x dilutions as shown in Table 13 below:

**Table 13**

| | ThrNeg | | ThrCurve | | ThrInvCurv |
|---|---|---|---|---|---|
| A | Thr neg | S1 | 1000.000 | S8 | 0.457 |
| B | Thr neg | S2 | 333.333 | S7 | 1.372 |
| C | Thr neg | S3 | 111.111 | S6 | 4.115 |
| D | Thr neg | S4 | 37.037 | S5 | 12.346 |
| E | Thr neg | S5 | 12.346 | S4 | 37.037 |
| F | Thr neg | S6 | 4.115 | S3 | 111.111 |
| G | Thr neg | S7 | 1.372 | S2 | 333.333 |
| H | Thr neg | S8 | 0.457 | S1 | 1000.000 |

• Make up Factor IXa as a control set of standards to run alongside the working assay, e.g. to make Standard 1 at 1000ng/ml FIXa add 0.638µl to 3ml diluent or buffer
• Pipette 80µl of each thrombin standard into the appropriate position of the assay plate. For example as shown in Table 14 below:

**Table 14**

| | Neg | Curve | Inv Curve | Neg | Curve | Inv Curve | Neg | Curve | Inv Curve | Neg | Curve | Inv Curve |
|---|---|---|---|---|---|---|---|---|---|---|---|---|
| A | neg | S1 | S8 | neg | S1 | S8 | neg | S1 | S8 | neg | S1 | S8 |
| B | neg | S2 | S7 | neg | S2 | S7 | neg | S2 | S7 | neg | S2 | S7 |
| C | neg | S3 | S6 | neg | S3 | S6 | neg | S3 | S6 | neg | S3 | S6 |
| D | neg | S4 | S5 | neg | S4 | S5 | neg | S4 | S5 | neg | S4 | S5 |
| E | neg | S5 | S4 | neg | S5 | S4 | neg | S5 | S4 | neg | S5 | S4 |
| F | neg | S6 | S3 | neg | S6 | S3 | neg | S6 | S3 | neg | S6 | S3 |
| G | neg | S7 | S2 | neg | S7 | S2 | neg | S7 | S2 | neg | S7 | S2 |
| H | neg | S8 | S1 | neg | S8 | S1 | neg | S8 | S1 | neg | S8 | S1 |

• Pipetting of multiple standards can be made easier by first transferring the total volume of each standard to one column in a deep well plate in rows A-H and then using an 8-channel pipettor to transfer the whole column of 80µl of each standard at a time to the assay plate, turning the assay plate upside-down when dispensing the inverted curve standards
• Prepare IL-6 negative and standard curves as previously described. One set should be used at the start and one at the end of each experiment to confirm chip functionality

### Incubate Aptamer Solution with Thrombin

- Once the bead/aptamer and the IL-6 bead/antibody solutions have rotated end-over-end for at least 5 minutes vortex for 10 secs to ensure maximum bead dispersion
- Dispense the beads into a solution basin and pipette 40µl of each bead solution into the appropriate well on the assay plate. Start with the IL-6 controls and vortex and dispense each separate bead lot at a time so that the beads do not settle and are kept moving as much as possible throughout this process
- Once all the bead solutions are added to the protein standards in the assay plate, place the plate on the Big Bear shaker, turn on and set at a speed of 1200 rpm
- Note the desired incubation times and ensure the Apta-ViBE is primed in time to run the samples at these times
- Start up the Apta-ViBE around 2 hours before the first column of samples is ready to be run

### 6. Run Samples Through the Apta-ViBE

### Aim

Detect thrombin in appropriate samples when they are loaded into the Apta-ViBE, run through the microfluidic channels and detected by the BioScale chip.

### Materials

- Corning 96 Well Assay Plate containing incubating samples
- 8 channel pipettor
- Running Buffer (degassed for at least 24 hours)
- Washing Buffer (degassed for at least 24 hours)
- Regeneration Buffer
- Solution Basins 55ml

### Method

### Prime Vibe

- At the start of the assay pull the window blind down to shield equipment and solutions from sun and possible temperature fluctuations and if possible control ambient temperature with a thermostat. Monitor surrounding temperature with thermometer throughout assay.
- Turn on the ViBE and open the Wiggler software on the PC
- Run the "Startup.txt" script and follow instructions on screen. This script will prompt the user to insert a BioScale cartridge, scan the barcode and enter information on the assay title. The ViBE will check for pressure leaks
- Run the "SensorConditioning.txt" script and follow the instructions on screen. This script will prompt the user to wet the cartridges with washing buffer twice, the transfer functions will be obtained and then the user will be prompted to add regeneration buffer 6 times
- If the ViBE is idle for more than 30 minutes after "SensorConditioning.txt" has finished then run "5mWetting.txt" script to wet the cartridge again before running samples

### Run Samples

- When the samples are ready to be run, execute "RegenerationAssay25sec.txt". This will prompt the user to add Washing Buffer, followed by Regeneration Buffer, followed by Washing Buffer again and finally 100µl of each sample. This script takes approximately 12 minutes from start to finish
- The results of each sample run are automatically stored in the C:BioScale/Data folder

### Shutdown ViBE

- Once the last set of samples have been run, execute the "Shutdown.txt" script and follow instructions on screen to flush out buffer and remove the BioScale cartridge
- Clean all surfaces with 70% ethanol
- Turn off ViBE and shut down the computer

### 7. Analyse Data

### Aim

To generate a thrombin calibration curve of concentration against signal - acoustic membrane microparticle detection (AMMP) response units (RU). Using the curve the limit of detection can be determined.

### Materials

- BioScale Data Analysis Excel Template

### Method

- Copy the master BioScale Excel results sheet and rename it appropriately for the experiment
- Copy the raw data from the C:BioScale/Data folder and paste it into the results tab of the Excel sheet
- Follow the instructions in the BioScale Excel results sheet entering all the appropriate information
- Click on the "analyze" button
- View the calibration curve with all the plotted data from the experiment.

### RESULTS

Initial experiments were undertaken to demonstrate the detection of thrombin in the ViBE bioanalyzer using a pair of aptamers against thrombin as capture molecules.

### Study 101110 - Thrombin + Tris-HCL + Salts

This experiment was carried out replacing incubation buffers (Aptamer mix diluent and binding buffer) with Tris-HCl + Salts Buffer (THS) (pH 7.4, 50mM Tris, 100mM NaCl, 5mM KCl, 1mM MgCl₂ and 5mM CaCl₂). This buffer is based on the selection buffer used during the original SELEX procedure that generated the 15mer aptamer (Bock *et al.* 1992). Thrombin incubation times went up to 2¼ hours. This was deemed sufficient as, although BioScale recommend incubation times of up to 3 or 4 hours for antibodies, all the published thrombin aptamer incubation times have been optimized at much shorter times.

### Protocol Components

| | |
|---|---|
| Bead Used | BioScale |
| Bead Storage Buffer | BioScale |
| B15Aptamer Orientation (and Purification) | 5' Biotin NEW (SePOP) |
| B15Aptamer Amount added per 1mg Beads | 200 picomoles |
| Bead-Apt Conjugation Buffer | PBS |
| Aptamer Mix Diluent | THS |
| Volume of beads with Optimum Accumulation | 40µl |
| Thrombin Dilutions | S1-S8 (2000ng/ml down to 0.009ng/ml) |
| F29Aptamer Orientation (and Purification) | 5' Fluor - IDT (HPLC) |
| Binding Buffer | THS |
| Incubation Times | 1-2¼ hours |
| Running Buffer | PBST + 10% BioScale Diluent |
| Wash Buffer | PBST |

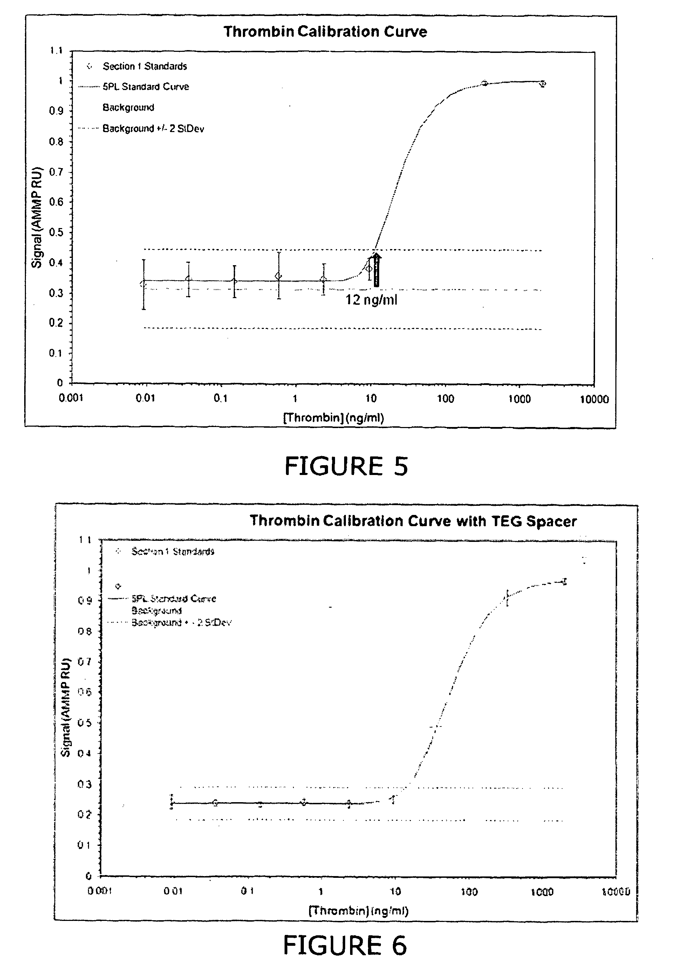

The results of this study are shown in Figure 5 wherein it can be seen that detection of thrombin was achieved at the two high concentrations of 2000 ng/ml and 333 ng/ml, establishing proof-of-concept, but not at lower concentrations.

Not only was a successful lower limit of detection (LLOD) for thrombin achieved, but this experiment validated the binding and running methodologies that were designed.

LLOD (where the curve reaches 2 Standard Deviations above background) is 12 ng/ml thrombin. One limitation of estimating this LLOD is that there are 6 data points which are near to background level and only 2 data points that exhibit detection, both of which are at the maximum limit of detection. To gain a better estimate, intermediate data points nearer the LLOD should be investigated.

One factor which may increase sensitivity of thrombin detection is the addition of a TEG spacer (a triethylene glycol 16-atom mixed polarity spacer) between the biotin molecule and the 15mer thrombin aptamer. This is because it is possible there is steric hindrance occurring due to the small length of aptamer which is required to bind both the magnetic bead and thrombin. The TEG spacer will confer a greater degree of flexibility within the bead-aptamer complex and may result in greater accessibility and binding to the chip surface, resulting in greater sensitivity. The next experiment will investigate the addition of a TEG spacer to determine the effects of possible decreased steric hindrance. As proof-of-concept has now been established the following experiments aim to optimize on the LLOD of thrombin. Parameters such as oligonucleotide spacers, incubation times and temperatures, aptamer concentrations and various buffers will be tested in turn in order to establish the optimized running conditions of the assay.

### Study 101115 - Thrombin B15TEG Spacer

This experiment was carried out replacing the initial biotinylated 15mer aptamer with the biotinylated-TEG-15 mer aptamer. TEG spacers require additional purification by reverse phase HPLC (HPLC-RP) after synthesis, compared to biotinylated oligos alone, 5' Biotin NEW, which require SePOP (Selective Precipitation Optimised Process) desalting.

### TEG Spacer Molecule

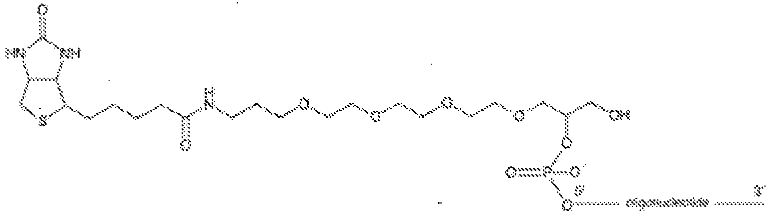

After conjugation of the aptamer to the bead, the bead count gave a volume of 50µl bead to add to each well for optimum accumulation, therefore this was the volume added to each well in the main experiment.
Due to the essential addition of the magnesium and other salts in the incubation buffer it was decided to add these to the running buffer and wash buffer too. Therefore the PBST (phosphate buffered saline with Tween) was replaced with THST (Tris-HCl + Salts Buffer with Tween).

### Protocol Components

| Bead Used | BioScale |
|---|---|
| Bead Storage Buffer | BioScale |
| B15Aptamer Orientation (and Purification) | 5' Biotin TEG (HPLC-RP) |
| B15Aptamer Amount added per 1mg Beads | 200 picomoles |
| Bead-Apt Conjugation Buffer | PBS |
| Aptamer Mix Diluent | THS |
| Volume of beads with Optimum Accumulation | 50µl |
| Thrombin Dilutions | S1-S8 + Extra run 333-2.6ng/ml dilutions at 3 hours |
| F29Aptamer Orientation (and Purification) | 5' Fluor - IDT (HPLC) |
| Binding Buffer | THS |
| Incubation Times | 1-3 hours |
| Running Buffer | THST + 10% BioScale Diluent |
| Wash Buffer | THST |

The results of this study are shown in Figure 6 wherein it can be seen that detection of thrombin was achieved at the two high concentrations of 2000 ng/ml and 333 ng/ml, but not at lower concentrations. LLOD is very similar to the previous experiment, therefore the addition of a spacer seems to have no effect on sensitivity of thrombin detection when compared to the original aptamer. Background is lower. Temperature may affect the quadruplex 3D structure of the thrombin aptamer.

### Study 101117 - Thrombin B15TEG at 37°C

This experiment was carried out with incubation of aptamers and thrombin at 37°C. This was achieved by placing the microplate shaker in a bench top incubator and monitoring the temperature throughout the experiment.
It is possible that additional blockers may be required to stop non-specific binding to the streptavidin beads and to the anti-fluorescein antibodies on the BioScale chip. When using antibodies the incubation medium is BioScale diluent (PBS with 10% fetal bovine serum, pH7) so adding fetal bovine serum to the THS may increase signal by reducing non-specific binding. Therefore, 10% BioScale diluent was also added to the THS incubation buffers as this is the same dilution as in the running buffer.

### Protocol Components

| | |
|---|---|
| Bead Used | BioScale |
| Bead Storage Buffer | BioScale |
| B15Aptamer Orientation (and Purification) | 5' Biotin TEG (HPLC-RP) |
| B15Aptamer Amount added per 1mg Beads | 200 picomoles |
| Bead-Apt Conjugation Buffer | PBS |
| Aptamer Mix Diluent | THS with 10% BioScale Diluent |
| Volume of beads with Optimum Accumulation | 50µl |
| Thrombin Dilutions | S1-S8 (2000ng/ml down to 0.009ng/ml) |
| F29Aptamer Orientation (and Purification) | 5' Fluor - IDT (HPLC) |
| Binding Buffer | THS with 10% BioScale Diluent |
| Incubation Times | 1-4 hours |
| Running Buffer | THST + 10% BioScale Diluent |
| Wash Buffer | THST |

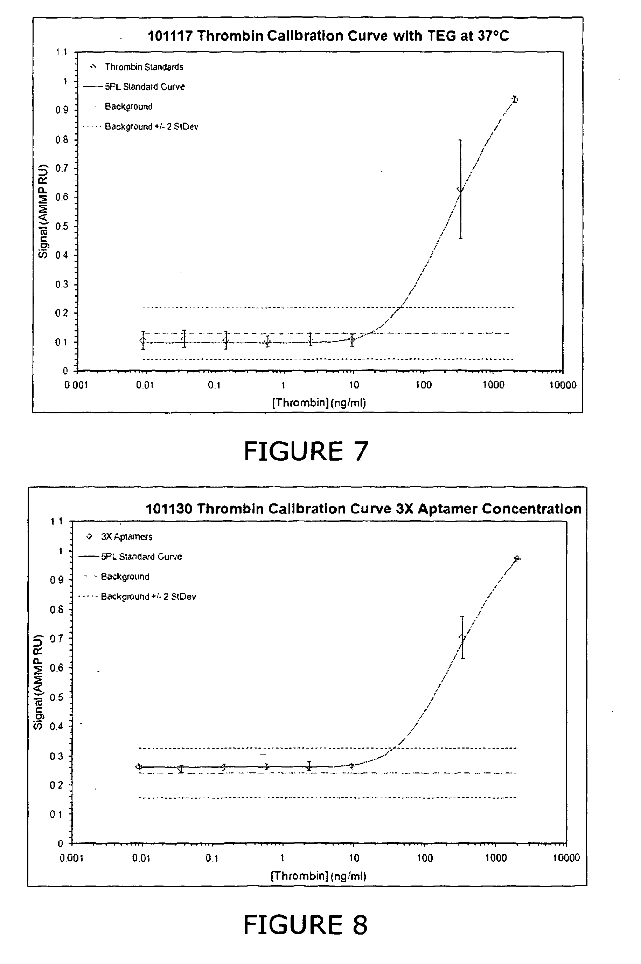

The results of this study are shown in Figure 7 wherein it can be seen that detection of thrombin was achieved at the two high concentrations of 2000 ng/ml and 333 ng/ml,although 333 ng/ml thrombin yielded a much reduced signal compared to the experiment at 22°C. Again there was no detection of the 6 lower thrombin concentrations. LLOD is 50 ng/ml. This may suggest that incubation at 37°C decreases the sensitivity of thrombin detection. However, a limitation of this assumption is that although the incubation is at 37°C, there is a period of a few minutes before the samples flow over the chip, additionally the running buffer, which is at ambient temperature) is introduced into the ViBE at this point and mixes with the samples so they are not kept at this temperature right to the end. A lower temperature may have an effect.

### Study 101130 - Thrombin B15TEG 3X Aptamers

This experiment was carried out using 3 times the amount (600 picomoles) of aptamer to the bead conjugation and of the F29 aptamer to the aptamer mix. After conjugation of the 3X aptamer to the bead, the bead count gave a volume of 30µl bead to add to each well for optimum accumulation, therefore this was the volume added to each well in the main experiment.

### Protocol Components

| | |
|---|---|
| Bead Used | BioScale |
| Bead Storage Buffer | BioScale |
| B15Aptamer Orientation (and Purification) | 5' Biotin TEG (HPLC-RP) |
| B15Aptamer Amount added per 1mg Beads | 600 picomoles 3X Aptamer Volume |
| Bead-Apt Conjugation Buffer | THS |
| Aptamer Mix Diluent | 10% of 10X THS in BioScale Diluent |
| Volume of beads with Optimum Accumulation | 30µl |
| Thrombin Dilutions | S1-S8, S9-S16 |
| F29Aptamer Orientation (and Purification) | 5' Fluor - IDT (HPLC) |
| Binding Buffer | 10% of 10X THS in BioScale Diluent |
| Incubation Times | 1-3½ hours |
| Running Buffer | THST + 10% BioScale Diluent |
| Wash Buffer | THST |

The results of this study are shown in Figure 8 wherein it can be seen that detection of thrombin was achieved at the two high concentrations of 2000 ng/ml and 333 ng/ml, but not at lower concentrations. LLOD is 40 ng/ml. Limitations are that this cannot be directly compared with the 1X aptamer as the experiments were done on different days.

### Study 101221 HEPES Buffer Experiment

This experiment was carried out using 4 different buffers:
1. THS;
2. HEPES-Saline (the optimum used in the Edwards (2010) paper);
3. HEPES-Saline + our salt combination - a hybrid of these 2;
4. HEPES-Saline(with 100mMNaCl) + our salt combination.
Thrombin incubation times were every 15 mins up to 1 hour. Due to the size of the experiment only 1 bead make could be used. As the 1X aptamer bead make gave the lower background, it was this one that was used for the experiment.

### Protocol Components

| Bead Used | BioScale |
|---|---|
| Bead Storage Buffer | BioScale |
| B15Aptamer Orientation (and Purification) | 5' Biotin TEG (HPLC-RP) |
| B15Aptamer Amount added per 1mg Beads | 200 picomoles of 1X |
| Bead-Apt Conjugation Buffer | THS |
| Aptamer Mix Diluent | Buffers 1, 2, 3 or 4 |
| Volume of beads with Optimum Accumulation | 40 µl of 1X Apt Beads |
| Thrombin Dilutions | S9-S16 |
| F29Aptamer Orientation (and Purification) | 5' Fluor - IDT (HPLC) |
| Binding Buffer | Buffers 1, 2, 3 or 4 |
| Incubation Times | 15 mins up to 1 hour |
| Running Buffer | THST + 10% BioScale Diluent |
| Wash Buffer | THST |

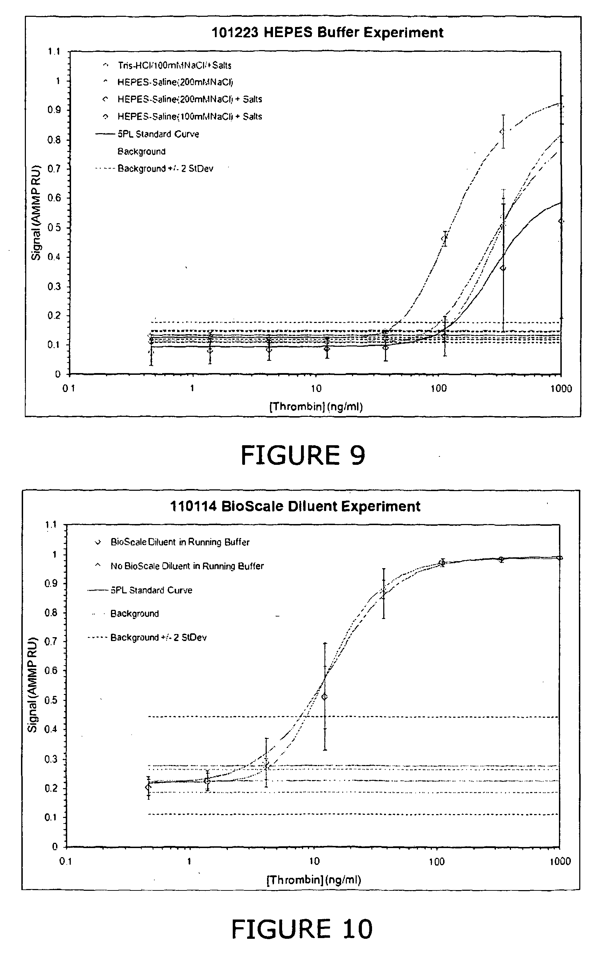

The results of this study are shown in Figure 9 wherein it can be seen that THS gave the best signal for thrombin detection compared to all 3 other buffers. Detection of thrombin was achieved with THS at the three higher concentrations of 1000 ng/ml and 333 ng/ml, and 111 ng/ml, but not at lower concentrations. LLOD with THS is 50 ng/ml thrombin. When comparing the two HEPES-Saline + Salts buffers, the effect of increased NaCl concentration (green curve - 200mM NaCl) compared to the lower NaCl concentration (black curve - 100mM NaCl), results in a better signal. Therefore, it may be advisable to increase the NaCl concentration in THS buffer to 200mM.

### Study 110114 Removal of BioScale Diluent

This experiment investigated the removal of the BioScale Diluent from the Running Buffer by comparing the normal Running Buffer (THST with 10% BioScale Diluent) with THST alone as the Running Buffer. The BioScale Diluent was also removed from the Aptamer Mix Diluent.

### Protocol Components

| Bead Used | BioScale |
|---|---|
| Bead Storage Buffer | BioScale |
| B15Aptamer Orientation (and Purification) | 5' Biotin TEG (HPLC-RP) |
| B15Aptamer Amount added per 1mg Beads | 200 picomoles of 1X |
| Bead-Apt Conjugation Buffer | THS |
| Aptamer Mix Diluent | THS |
| Volume of beads with Optimum Accumulation | 271.62µl beads in 3ml diluent - from bead count |
| Thrombin Dilutions | S9-S16 |
| F29Aptamer Orientation (and Purification) | 5' Fluor - IDT (HPLC) |
| Binding Buffer | THS |
| Incubation Times | 20, 40, 60, 80 mins |
| Running Buffer | THST + or - 10% BioScale Diluent |
| Wash Buffer | THST |

The results of this study are shown in Figure 10 wherein it can be seen that using the Running Buffer without BioScale Diluent resulted in a LLOD of 4 ng/ml thrombin. The calibration curves of thrombin using both Running Buffers were very similar, however, the background and variability of the data were higher when using the Running Buffer that contained 10% BioScale Diluent, so as a consequence the LLOD was higher at 7.5 ng/ml.
The assay using BioScale Diluent in the Running Buffer in this experiment is comparable to those which gave a LLOD of 12 ng/ml (see Studies 101110 and 101115). However, the calibration curves for those assays were based on thrombin concentrations that were very high from or much lower than the LLOD. Using the intermediate thrombin concentrations, which are distributed much closer to the LLOD in this experiment results in a calibration curve of which the LLOD is a much more reliable estimate.

It appears then, that removal of the BioScale Diluent from the Running Buffer is more optimal for thrombin detection in this assay and the current level of thrombin detection is 4 ng/ml. However, the experiment will need to be repeated in triplicate in order to fully establish this parameter.

### Conclusions

Aptamers have been successfully adapted for use with the BioScale ViBE. The proof of concept has been demonstrated using human alpha-thrombin (as detailed in Study 110114 Removal of BioScale Diluent) The LLOD of 4 ng/ml has been shown to be the most sensitive level of detection currently available for the ViBE using aptamers against a protein target.

## Claims

1. A method of detecting the presence of an analyte within a sample,
wherein said method comprises the steps of:
(a) preparing one or more magnetic complexes comprising a magnetic particle coated with a first binding agent capable of specific binding to the analyte, wherein a spacer element is present between the first binding agent and the magnetic particle;
(b) adding the one or more magnetic complexes prepared in step (a) to the sample within a receptacle, wherein a surface of a receptacle is coated with a second binding agent capable of specific binding to the analyte, such that at least one of said first and second binding agents is a DNA or RNA oligonucleotide;
(c) temporarily applying a magnetic field to the surface of the receptacle which is coated with the second binding agent; and
(d) detecting the presence of magnetic complexes which remain bound to the surface of the receptacle following step (c) which is indicative of the presence of the analyte within the sample.

2. A method as defined in claim 1, wherein the first and second binding agents are identical, such as both the first and second binding agents are DNA or RNA oligonucleotides, or wherein the first binding agent differs from the second binding agent, such as both the first and second binding agents are both DNA or RNA oligonucleotides having differing sequences, or one of the first and second binding agents is a DNA or RNA oligonucleotide and the other is an antibody.

3. A method as defined claim 1 or claim 2, wherein the first binding agent is directly bound to the magnetic particle.

4. A method as defined in any of claims 1 to 3, wherein the first binding agent is indirectly bound to the magnetic particle.

5. A method as defined in any of claims 1 to 4, wherein the first and second members of the binding pair comprise biotin and avidin or biotin and derivatives of avidin, such as streptavidin and neutravidin, or wherein the first and second members of the binding pair comprise an anti-fluorescein antibody and fluorescein, such as wherein the second binding agent is fluoresceinated and the receptacle is coated with an anti-fluorescein antibody.

6. A method as defined in any of claims 1 to 5, wherein the spacer element is a triethylene glycol (TEG) spacer element, such as a triethylene glycol 16-atom mixed polarity spacer.

7. A method as defined in any of claims 1 to 6, wherein the surface of the receptacle comprises a sensing device such as a flexural plate wave (FPW) device or a flow cytometer.

8. A method as defined in any of claims 1 to 7, wherein the detection step (d) is performed after the magnetic field has been removed.

9. A method as defined in any of claims 1 to 8, wherein the sample is a biological sample, such as blood, saliva, sputum, plasma, serum, ocular lens fluid, cerebrospinal fluid, sweat, urine, milk, ascites fluid, synovial fluid, peritoneal fluid, amniotic fluid and fecal matter.

10. A method as defined in any of claims 1 to 9, wherein the analyte is a biological analyte, such as a polypeptide, a nucleic acid, a carbohydrate, a nucleoprotein, a glycopeptide or a glycolipid or wherein the analyte is a pathogen or microbe, such as bacteria (for example *Staphylococcus aureus or Clostridium difficile*) or bacterial spores, viruses (such as Influenza), parasites, prions or other pathogens (such as Giardia, malaria and cryptosporidia) or their cell wall or surface components such as gram-positive peptidoglycans, lipoteichoic and teichoicacids, and gram- negative endotoxin (e.g. lipopolysaccharide), or a small molecule, or sortase B or protein A of *Staphylococcus aureus,* or toxin A or toxin B of *Clostridium difficile*, or the M2e peptide of Influenza.

11. A method of diagnosing or monitoring a disease within a subject, wherein said diagnostic method comprises the steps of:
(a) preparing one or more magnetic complexes comprising a magnetic particle coated with a first binding agent capable of specific binding to an analyte biomarker which is characteristic for the disease, wherein a spacer element is present between the first binding agent and the magnetic particle;
(b) adding the one or more magnetic complexes prepared in step (a) to a biological sample obtained from the subject within a receptacle, wherein a surface of a receptacle is coated with a second binding agent capable of specific binding to the analyte biomarker which is characteristic for the disease, such that at least one of said first and second binding agents is a DNA or RNA oligonucleotide;
(c) temporarily applying a magnetic field to the surface of the receptacle which is coated with the second binding agent; and
(d) detecting the presence of magnetic complexes which remain bound to the surface of the receptacle following step (c) which is indicative of the presence of the analyte biomarker characteristic for the disease within the biological sample, or a binding value which is above or below a threshold value characteristic for the disease may be indicative of presence of the disease or risk for the disease.

12. A method as defined in claim 11, wherein diagnosis is intended to detect the presence of bacteria, a virus, cancer, an autoimmune disease, a cardiovascular disease, a degenerative disorder or a psychiatric disorder.

13. A magnetic analyte detection complex which comprises a second binding agent - analyte - first binding agent - magnetic particle complex bound to the surface of a receptacle via interaction between a first member of a binding pair present on the surface of the receptacle and a second member of a binding pair present on the second binding agent, wherein a spacer element is present between the first binding agent and the magnetic particle.

14. A method for determining the quantity of analyte in a sample which comprises the steps of forming a surface bound complex as defined in claim 13 and quantifying the amount of the complex present on said surface to determine the quantity of analyte in said sample.

## Patentansprüche

1. Verfahren zum Nachweisen der Anwesenheit eines Analyten in einer Probe, wobei das genannte Verfahren die folgenden Schritte beinhaltet:
(a) Herstellen von einem oder mehreren magnetischen Komplexen, die einen magnetischen Partikel umfassen, der mit einem ersten Bindemittel beschichtet ist, das sich speziell an den Analyt binden kann, wobei ein Abstandselement zwischen dem ersten Bindemittel und dem magnetischen Partikel vorhanden ist;
(b) Zugeben der in Schritt (a) hergestellten ein oder mehreren magnetischen Komplexe zu der Probe in einer Aufnahme, wobei eine Oberfläche einer Aufnahme mit einem zweiten Bindemittel beschichtet ist, das sich speziell an den Analyt binden kann, so dass wenigstens eines der genannten ersten und zweiten Bindemittel ein DNA- oder RNA-Oligonukleotid ist;
(c) vorübergehendes Anlegen eines Magnetfeldes an die mit dem zweiten Bindemittel beschichtete Oberfläche der Aufnahme; und
(d) Nachweisen der Anwesenheit von magnetischen Komplexen, die nach Schritt (c) an der Oberfläche der Aufnahme gebunden bleiben, was die Anwesenheit des Analyten in der Probe anzeigt.

2. Verfahren nach Anspruch 1, wobei das erste und zweite Bindemittel identisch sind, so dass sowohl das erste als auch das zweite Bindemittel DNA-oder RNA-Oligonukleotide sind, oder wobei sich das erste Bindemittel von dem zweiten Bindemittel so unterscheidet, dass sowohl das erste als auch das zweite Bindemittel DNA- oder RNA-Oligonukleotide mit unterschiedlichen Sequenzen sind oder eines aus erstem und zweitem Bindemittel ein DNA- oder RNA-Oligonukleotid und das andere ein Antikörper ist.

3. Verfahren nach Anspruch 1 oder Anspruch 2, wobei das erste Bindemittel direkt an den magnetischen Partikel gebunden ist.

4. Verfahren nach einem der Ansprüche 1 bis 3, wobei das erste Bindemittel indirekt an den magnetischen Partikel gebunden ist.

5. Verfahren nach einem der Ansprüche 1 bis 4, wobei das erste und zweite Element des Bindungspaars Biotin und Avidin oder Biotin und Avidinderivate wie Streptavidin und Neutravidin umfassen, oder wobei das erste und zweite Element des Bindungspaares einen Anti-Fluoreszein-Antikörper und Fluoreszein umfassen, z.B. so dass das zweite Bindemittel fluoresziert und die Aufnahme mit einem Anti-Fluoreszein-Antikörper beschichtet ist.

6. Verfahren nach einem der Ansprüche 1 bis 5, wobei das Abstandselement ein TEG-(Triethylenglykol)-Abstandselement wie z.B. ein Triethylenglykol-Abstandselement mit 16-Atome-Mischpolarität ist.

7. Verfahren nach einem der Ansprüche 1 bis 6, wobei die Oberfläche der Aufnahme eine Erfassungsvorrichtung wie z.B. eine FPW-(Biegeplattenwellen)-Vorrichtung oder ein Durchflusszytometer ist.

8. Verfahren nach einem der Ansprüche 1 bis 7, wobei der Nachweisschritt (d) nach dem Wegnehmen des Magnetfelds ausgeführt wird.

9. Verfahren nach einem der Ansprüche 1 bis 8, wobei die Probe eine biologische Probe wie z.B. Blut, Speichel, Sputum, Plasma, Serum, Augenlinsenfluid, Rückenmarksflüssigkeit, Schweiß, Urin, Milch, aszite Flüssigkeit, Gelenkflüssigkeit, peritoneale Flüssigkeit, Fruchtwasser und Fäkalien ist.

10. Verfahren nach einem der Ansprüche 1 bis 9, wobei der Analyt ein biologischer Analyt ist, wie z.B. ein Polypeptid, eine Nukleinsäure, ein Kohlenhydrat, ein Nukleoprotein, ein Glykopeptid oder ein Glykolipid, oder wobei der Analyt ein Pathogen oder eine Mikrobe ist, wie z.B. Bakterien (z.B. *Staphylococcus aureus oder Clostridium difficile*) oder bakterielle Sporen, Viren (z.B. Influenza), Parasiten, Prionen oder andere Pathogene (wie z.B. Giardia, Malaria und Cryptosporidia) oder deren Zellwand- oder Oberflächenkomponenten, wie grampositive Peptidoglykane, Lipoteichon- oder Teichonsäure, und gramnegatives Endotoxin (z.B. Lipopolysaccharid), oder ein kleines Molekül oder Sortase B oder Protein A von *Staphylococcus aureus,* oder Toxin A oder Toxin B von *Clostridium difficile*, oder das M2e-Peptid von Influenza.

11. Verfahren zum Diagnostizieren oder Überwachen einer Krankheit in einem Subjekt, wobei das genannte Diagnoseverfahren die folgenden Schritte beinhaltet:
(a) Herstellen von einem oder mehreren magnetischen Komplexen, die einen magnetischen Partikel umfassen, der mit einem ersten Bindemittel beschichtet ist, das sich speziell an einen Analyt-Biomarker binden kann, der für die Krankheit charakteristisch ist, wobei ein Abstandselement zwischen dem ersten Bindemittel und dem magnetischen Partikel vorhanden ist;
(b) Zugeben der in Schritt (a) hergestellten ein oder mehreren magnetischen Komplexe zu einer von dem Subjekt erhaltenen biologischen Probe in einer Aufnahme, wobei eine Oberfläche einer Aufnahme mit einem zweiten Bindemittel beschichtet ist, das sich speziell an den Analyt-Biomarker binden kann, der für die Krankheit charakteristisch ist, so dass wenigstens eines der genannten ersten und zweiten Bindemittel ein DNA- oder RNA-Oligonukleotid ist;
(c) vorübergehendes Anlegen eines Magnetfeldes an die mit dem zweiten Bindemittel beschichtete Oberfläche der Aufnahme; und
(d) Nachweisen der Anwesenheit von magnetischen Komplexen, die nach Schritt (c) an der Oberfläche der Aufnahme gebunden bleiben, was die Anwesenheit des für die Krankheit charakteristischen Analyt-Biomarkers in der biologischen Probe anzeigt, oder ein Bindewert, der über oder unter einem für die Krankheit charakteristischen Schwellenwert liegt, kann die Anwesenheit der Krankheit oder eines Risikos für die Krankheit anzeigen.

12. Verfahren nach Anspruch 11, wobei mit der Diagnose die Anwesenheit von Bakterien, eines Virus, Krebs, einer Autoimmunkrankheit, einer Herz-Kreislauf-Krankheit, einer degenerativen Störung oder einer psychiatrischen Störung erkannt werden soll.

13. Komplex zum Nachweisen eines magnetischen Analyten, der einen Komplex aus zweites Bindemittel - Analyt - erstes Bindemittel - Magnetpartikel umfasst, gebunden an die Oberfläche einer Aufnahme per Interaktion zwischen einem ersten Element eines auf der Oberfläche der Aufnahme vorhandenen Bindungspaares und einem zweiten Element eines auf dem zweiten Bindemittel vorhandenen Bindungspaares, wobei ein Abstandselement zwischen dem ersten Bindemittel und dem magnetischen Partikel vorhanden ist.

14. Verfahren zum Ermitteln der Analytmenge in einer Probe, das die Schritte des Bildens eines oberflächengebundenen Komplexes nach Anspruch 13 und des Quantifizierens der Menge des auf der genannten Oberfläche vorhandenen Komplexes beinhaltet, um die Analytmenge in der genannten Probe zu bestimmen.

## Revendications

1. Méthode permettant de détecter la présence d'un analyte dans un échantillon, la dite méthode comprenant les étapes :
(a) de préparation de un ou plusieurs complexes magnétiques comprenant une particule magnétique enduite avec un premier agent de liaison capable de se lier spécifiquement à l'analyte, où un élément espaceur est présent entre le premier agent de liaison et la particule magnétique ;
(b) d'addition du un ou des plusieurs complexes magnétiques préparés à l'étape (a) à l'échantillon dans un réceptacle, où une surface d'un réceptacle est enduite avec un second agent de liaison capable de se lier spécifiquement à l'analyte, de sorte qu'au moins un desdits premier et second agents de liaison est un oligonucléotide d'ADN ou d'ARN ;
(c) d'application temporaire d'un champ magnétique à la surface du réceptacle qui est enduite avec le second agent de liaison ; et
(d) de détection de la présence de complexes magnétiques qui demeurent liés à la surface du réceptacle après l'étape (c), qui est indicative de la présence de l'analyte dans l'échantillon.

2. Méthode selon la revendication 1, dans laquelle les premier et second agents de liaison sont identiques si bien que les premier et second agents de liaison sont tous deux des oligonucléotides d'ADN ou d'ARN, ou dans laquelle le premier agent de liaison diffère du second agent de liaison si bien que les premier et second agents de liaison sont tous deux des oligonucléotides d'ADN ou d'ARN de séquences différentes, ou l'un des premier et second agents de liaison est un oligonucléotide d'ADN ou d'ARN et l'autre est un anticorps.

3. Méthode selon la revendication 1 ou la revendication 2, dans laquelle le premier agent de liaison est directement lié à la particule magnétique.

4. Méthode selon l'une quelconque des revendications 1 à 3, dans laquelle le premier agent de liaison est indirectement lié à la particule magnétique.

5. Méthode de l'une quelconque des revendications 1 à 4, dans laquelle les premier et second composants de la paire de liaison comprennent la biotine et l'avidine ou la biotine et des dérivés de l'avidine comme la streptavidine et la neutravidine, ou dans laquelle les premier et second composants de la paire de liaison comprennent un anticorps anti-fluorescéine et la fluorescéine, p. ex. quand le second agent de liaison est conjugué à fluorescéine et le réceptacle enduit avec un anticorps anti-fluorescéine.

6. Méthode de l'une quelconque des revendications 1 à 5, dans laquelle l'élément espaceur est un élément espaceur à base de triéthylène-glycol (TEG) comme un espaceur à base de triéthylène-glycol à 16 atomes de polarité mixte.

7. Méthode selon l'une quelconque des revendications 1 à 6, dans laquelle la surface du réceptacle comprend un dispositif capteur comme un dispositif à onde de plaque flexible ou un cytomètre à flux.

8. Méthode selon l'une quelconque des revendications 1 à 7, dans laquelle l'étape de détection (d) est effectuée après le retrait du champ magnétique.

9. Méthode selon l'une quelconque des revendications 1 à 8, dans laquelle l'échantillon est un échantillon biologique comme le sang, la salive, une expectoration, le plasma, le sérum, l'humeur aqueuse, le liquide céphalorachidien, la sueur, l'urine, le lait, le liquide ascitique, le liquide synovial, le liquide péritonéal, le liquide amniotique et les matières fécales.

10. Méthode selon l'une quelconque des revendications 1 à 9, dans laquelle l'analyte est un analyte biologique comme un polypeptide, un acide nucléique, un hydrate de carbone, une nucléoprotéine, un glycopeptide ou un glycolipide, ou dans laquelle l'analyte est un agent pathogène ou un microbe comme des bactéries (par exemple *Staphylococcus aureus* ou *Clostridium difficile*) ou des spores bactériennes, des virus (comme le virus de la grippe), des parasites, des prions, d'autres pathogènes (comme ceux responsables de la giardiose, du paludisme ou de la cryptosporidiose) ou des composants de leur paroi ou surface cellulaire, par exemple les peptidoglycanes et les acides lipotéichoïque et téichoïque d'une bactérie Gram positif et l'endotoxine (p. ex. un lipopolysaccharide) d'une bactérie Gram négatif ou une petite molécule, ou la sortase B ou la protéine A de *Staphylococcus aureus* ou la toxine A ou la toxine B de *Clostridium difficile* ou le peptide M2e du virus de la grippe.

11. Méthode de diagnostic ou de suivi d'une maladie chez un sujet, ladite méthode de diagnostic comprenant les étapes :
(a) de préparation de un ou plusieurs complexes magnétiques comprenant une particule magnétique enduite avec un premier agent de liaison capable de se lier spécifiquement à un analyte biomarqueur qui est caractéristique de la maladie, où un élément espaceur est présent entre le premier agent de liaison et la particule magnétique ;
(b) d'addition du un ou des plusieurs complexes magnétiques préparés à l'étape (a) à un échantillon biologique obtenu chez le sujet et placé dans un réceptacle, où une surface du réceptacle est enduite avec un second agent de liaison capable de se lier spécifiquement à l'analyte biomarqueur caractéristique de la maladie, de sorte qu'au moins un desdits premier et second agents de liaison est un oligonucléotide d'ADN ou d'ARN ;
(c) d'application temporaire d'un champ magnétique à la surface du réceptacle qui est enduite avec le second agent de liaison ; et
(d) de détection de la présence de complexes magnétiques qui demeurent liés à la surface du réceptacle après l'étape (c), qui est indicative de la présence de l'analyte biomarqueur caractéristique de la maladie dans l'échantillon biologique ou de détection d'un niveau de liaison qui est en dessus ou en dessous d'une valeur seuil caractéristique de la maladie, qui peut être indicatrice de la présence de la maladie ou d'un risque pour cette maladie.

12. Méthode selon la revendication 11, dans laquelle le diagnostic vise à détecter la présence d'une bactérie, d'un virus, d'un cancer, d'une maladie autoimmune, d'une maladie cardio-vasculaire, d'une affection dégénérative ou d'une affection psychiatrique.

13. Complexe magnétique de détection d'un analyte qui comprend un complexe « second agent de liaison - analyte - premier agent de liaison - particule magnétique » lié à la surface d'un réceptacle par le biais d'une interaction entre un premier composant d'une paire de liaison présente à la surface du réceptacle et un second composant d'une paire de liaison présente sur le second agent de liaison, où un élément espaceur est présent entre le premier agent de liaison et la particule magnétique.

14. Méthode permettant de déterminer la quantité d'un analyte dans un échantillon, qui comprend les étapes consistant à former un complexe lié à une surface selon la revendication 13 et la quantification du complexe présent sur ladite surface pour déterminer la quantité d'analyte dans ledit échantillon.


### SEQUENCE LISTING

<110> LOXBRIDGE RESEARCH LLP
<120> OLIGONUCLEOTIDE BASED ANALYTE DETECTION METHOD
<130> LOX-C-P1060PCT
<160> 34
<170> PatentIn version 3.5
<210> 1
   <211> 60
   <212> DNA
   <213> Synthetic
<400> 1
   cggaattccg cgtgtgccac cttttttgaa aatacatttg gtggtccgtt cgggatcctg 60
<210> 2
   <211> 60
   <212> DNA
   <213> Synthetic
<400> 2
   cggaattccg cgtgtgccac caaagttgtt gtttttcgag gtggtccgtt cgggatcctg 60
<210> 3
   <211> 60
   <212> DNA
   <213> Synthetic
<400> 3
   cggaattccg cgtgtgccac ctttgaattt atttttcctg gtggtccgtt cgggatcctg 60
<210> 4
   <211> 67
   <212> DNA
   <213> Synthetic
<400> 4
   cggaattccg cgtgtgccac cactgggggg ggttcgcatt gactggggtg gtccgttcgg 60
   gatcctg 67
<210> 5
   <211> 67
   <212> DNA
   <213> Synthetic
<400> 5
   cggaattccg cgtgtgccac cgggttatca gctttttagg tgacccggtg gtccgttcgg 60
   gatcctg 67
<210> 6
   <211> 67
   <212> DNA
   <213> Synthetic
<400> 6
   cggaattccg cgtgtgccac cgggtcctag tcatttaaat caacccggtg gtccgttcgg 60
   gatcctg 67
<210> 7
   <211> 67
   <212> DNA
   <213> Synthetic
<400> 7
   cggaattccg cgtgtgccac cggaacggag cacaacaggc ccagccggtg gtccgttcgg 60
   gatcctg 67
<210> 8
   <211> 67
   <212> DNA
   <213> Synthetic
<400> 8
   cggaattccg cgtgtgccac cttttcaacc tgcaaacccc caggaaggtg gtccgttcgg 60
   gatcctg 67
<210> 9
   <211> 60
   <212> DNA
   <213> Synthetic
<400> 9
   cggaattccg cgtgtgccac ctcgtttaac aaactcttag gtggtccgtt cgggatcctg 60
<210> 10
   <211> 60
   <212> DNA
   <213> Synthetic
<400> 10
   cggaattccg cgtgtgccac ctggtttctg gttgtcccgg gtggtccgtt cgggatcctg 60
<210> 11
   <211> 67
   <212> DNA
   <213> Synthetic
<400> 11
   cggaattccg cgtgtgccac cccgttggcg gctttcgacc ggtgggggtg gtccgttcgg 60
   gatcctg 67
<210> 12
   <211> 60
   <212> DNA
   <213> Synthetic
<400> 12
   cggaattccg cgtgtgccac cgcttttgag gcgcagcacg gtggtccgtt cgggatcctg 60
<210> 13
   <211> 60
   <212> DNA
   <213> Synthetic
<400> 13
   cggaattccg cgtgtgccac ctttttcggg ctttttcccg gtggtccgtt cgggatcctg 60
<210> 14
   <211> 60
   <212> DNA
   <213> Synthetic
<400> 14
   cggaattccg cgtgtgccac cggcgggaag attaacgcag gtggtccgtt cgggatcctg 60
<210> 15
   <211> 60
   <212> DNA
   <213> Synthetic
<400> 15
   cggaattccg cgtgtgccac ccaaacgatc caatgctagg gtggtccgtt cgggatcctg 60
<210> 16
   <211> 60
   <212> DNA
   <213> Synthetic
<400> 16
   cggaattccg cgtgtgccac ctaacaattt tagtttttgg gtggtccgtt cgggatcctg 60
<210> 17
   <211> 60
   <212> DNA
   <213> Synthetic
<400> 17
   cggaattccg cgtgtgccac cttggttttt cggcttcgtg gtggtccgtt cgggatcctg 60
<210> 18
   <211> 66
   <212> DNA
   <213> Synthetic
<400> 18
   cggaattccg cgtgtgccac cccattttat ccttccacca aggggggtgg tccgttcggg 60
   atcctg 66
<210> 19
   <211> 63
   <212> DNA
   <213> Synthetic
<400> 19
   cggaattccg cgtgtgccac ccacgccctt gttacctgtt aaggtggtcc gttcgggatc 60
   ctg 63
<210> 20
   <211> 67
   <212> DNA
   <213> Synthetic
<400> 20
   cggaattccg cgtgtgccac ctgctaacgt ctaattgaac ttcgggggtg gtccgttcgg 60
   gatcctg 67
<210> 21
   <211> 67
   <212> DNA
   <213> Synthetic
<400> 21
   cggaattccg cgtgtgccac ccgatttgtt agtgcggatt cagcccggtg gtccgttcgg 60
   gatcctg 67
<210> 22
   <211> 42
   <212> DNA
   <213> Synthetic
<400> 22
   attctatgac tacctacacc tcactgtctc tccacctttt tg 42
<210> 23
   <211> 42
   <212> DNA
   <213> Synthetic
<400> 23
   cacccgaccg aacttctctt tttcctacat atagcatcgt aa 42
<210> 24
   <211> 41
   <212> DNA
   <213> Synthetic
<400> 24
   acccacgaat ttccacacac tatcctatct ccactaatct a 41
<210> 25
   <211> 42
   <212> DNA
   <213> Synthetic
<400> 25
   ccctaacaaa catgtcaatt cagagatttt tacctaacat gc 42
<210> 26
   <211> 42
   <212> DNA
   <213> Synthetic
<400> 26
   gaacattaac actcgccgga atattccaac taccttttac ct 42
<210> 27
   <211> 42
   <212> DNA
   <213> Synthetic
<400> 27
   gtttggattg taccaccatt caattaactt actatactat ta 42
<210> 28
   <211> 42
   <212> DNA
   <213> Synthetic
<400> 28
   tcccatacca cattcccttc tcaaactatc aaaagctcag gg 42
<210> 29
   <211> 42
   <212> DNA
   <213> Synthetic
<400> 29
   caccacactt ttcctcattc aactaacgtt cgtcaccgca at 42
<210> 30
   <211> 42
   <212> DNA
   <213> Synthetic
<400> 30
   taacaatttc ttttacttcc atttccttat gcactaaatc tc 42
<210> 31
   <211> 41
   <212> DNA
   <213> Synthetic
<400> 31
   aacactcttt tctttattta ttgtctcttt tacttttttt t 41
<210> 32
   <211> 41
   <212> DNA
   <213> Synthetic
<400> 32
   tacacctttt tttaatccct tacatttacc attctttatc a 41
<210> 33
   <211> 42
   <212> DNA
   <213> Synthetic
<400> 33
   actttcctta ttatctttcc ctattttttg tttctattct at 42
<210> 34
   <211> 42
   <212> DNA
   <213> Synthetic
<400> 34
   ttttactcat cccaattacc tcttttctaa taactcgccc tt 42